# EUROPEAN PATENT SPECIFICATION

(11) **EP 2 352 876 B1**
(45) Date of publication and mention of the grant of the patent: **12.12.2012**
(21) Application number: 09740336.4
(22) Date of filing: 27.10.2009
(51) Int. Cl.: D06M 15/227, D06M 15/263, D06M 15/564, D06M 16/00, C09D 5/16, A01N 25/00, A01N 25/10, A01N 25/34

(54) **TREATED TEXTILE MATERIAL FOR USE IN AQUATIC ENVIRONMENTS**
BEHANDELTES TEXTILMATERIAL ZUM EINSATZ IN WÄSSRIGER UMGEBUNG
MATÉRIAU TEXTILE TRAITÉ DESTINÉ À ÊTRE UTILISÉ DANS DES ENVIRONNEMENTS AQUATIQUES

(30) Priority: 04.11.2008 EP 08168285
(43) Date of publication of application: 10.08.2011
(73) Proprietor: BASF SE, 67056 Ludwigshafen (DE)
(72) Inventor: KARL, Ulrich, 67269 Grünstadt (DE)
(86) International application number: PCT/EP2009/064132
(87) International publication number: WO 2010/052153

(56) References cited:
- WO-A1-2008/151984
- WO-A2-2006/128870
- DE-A1- 19 644 224
- US-A- 5 990 043

## Description

The invention relates to the use of a textile material treated with an antifouling agent in aquatic environments, e.g. in fishery or aquaculture.

Biofouling is the unwanted accumulation of microorganisms, plants and animals on man-made surfaces submersed into fresh water or sea water. Biofouling can decrease the heat exchange properties and flow properties of technical installations and promote corrosion through the release of strong organic acids.

Biofouling in aquaculture, specifically in mariculture is a problem because
- it decreases the oxygen in the water inside the net cages;
- it decreases the water flow reducing the natural food supply thus decreasing the growth rate of farmed finfish or shellfish;
- fouling organisms compete with cultured animals for nutrients supply;
- fouling organisms may harbour diseases or predators or release toxic substances;
- it physically damages equipment (abrasion, brittleness, increased weight).

Changing nets (removal from the water, dry in the air and clean in net washing machines) or cleaning in situ (high-pressure cleaners) is expensive:
- manual labour (especially divers are expensive);
- removing nets or cleaning poses stress on fish (2 % mortality; several days without growth);
- increased escapes of fish;
- if nets are washed in separate washing facilities, they need to be disinfected in order to avoid spreading infections.

The most common fouling species at finfish and shellfish production sites are: Algae, barnacles, mussels, tubeworms, ascidians and hydroids.

The early stages of succession in settlement (biofouling) are characterized by the prevalence of fast-growing species, such as hydroids, cirrepedes (e.g., barnacles), polychaetes, bryozoans and ascidians. Often the first settlers are bacteria, algae and hydroids, followed by other sessile aquatic organisms. But in many cases, barnacles and/or ascidians may be the first to attach to a surface. The speed and extent of settlement is largely determined by the biomass and diversity of fouling species present in the deep-sea or coastal or estuarine waters, as well as by the abundance of food, growth factors, water pollutants, abrasive matter (such as sand in the tidal zone), water temperature and strong or weak water currents. These factors may also lead to fouling which is dominated by one or a few of the organisms cited above.

Several attempts have been made to prevent the biofouling of textile materials, in particular nets that are used in aquatic environments, by impregnating the textile material with an antifouling agent.

JP-A 04-142373 discloses the use of silicone rubber compositions containing linear organo-polysiloxane containing polyoxyalkylene groups for coating fish nets.

US 4,883,852 discloses an antifouling coating comprising poly-dimethylsiloxane alkyl (meth)acrylate, optionally with other vinyl polymers. The general disadvantage of all polysiloxane coatings is that any settlement of aquatic organisms can only be removed if the surface is moved at a higher speed than usually found with aquaculture installations.

JP-A 04-225904 discloses a "dissolution control of antifouling agent" by treating fibres or ropes with one or more hydrophilic polymers prepared from ethoxylated (meth)acrylic ester monomers.

JP-A 01-061404 discloses underwater antifouling compositions containing inorganic peroxide. The disadvantage of this invention is the short duration of peroxide release because the peroxide is consumed by many other substances present in sea water.

JP-A 60-094677 (Kuraray KK) discloses an antifouling treatment of organic fibres by flame spraying them with metals such as copper, tin, cadmium, zinc, lead, bismuth, antimony, chromium, mercury and beryllium. These metals are obviously toxic to all aquatic organisms, and are, therefore, environmentally hostile.

JP-A 2002-069360 discloses an antifouling paint composition containing (A) a (meth)acrylate copolymer containing (a) (a-1) units of the aromatic vinyl compound (preferred: styrene) and (a-2) units of the alkyl (meth)acrylate component and (b) units of the hydroxyalkyl (meth)acrylate component and (B) an organic antifouling agent.

WO 2005/030405 discloses the use of block/random copolymers in biocidal compositions by e.g. covalently bonding a biocide such as trichlosan (converting its hydroxyl function into an ether link). Whilst the hydrolysis of the covalent bond may release the biocide over time, the chemical modification creates a new biocide which requires completely new registration.

JP-A 2006-188453 discloses an antifouling agent for coating fishing nets based on a biocide and certain low molecular weight (meth)acrylates.

In spite of the many proposals for antifouling compositions in the literature there is still ample room for improvement, in particular with respect to the antifouling efficacy and endurance of treated textile materials.

It has now been found that certain polymeric binders are particularly useful for impregnating textile materials that are used in aquatic applications.

Accordingly, in one aspect of the invention there is provided the use in an aquatic environment of a textile material, treated with an antifouling agent, said antifouling agent comprising
a) one or more organic antifouling biocides;
b) a polymeric binder, selected from the group consisting of
   (A) a polyethylenic binder with an average molecular mass Mₙ of 1500 to 20000 g/mol, obtainable from the following monomers
      (A1) from 60 to 95 % by weight of ethylene,
      (A2) from 5 to 40 % by weight of at least one unsaturated carboxylic acid, selected from the group of
         (A2a) monoethylenically unsaturated C₃-C₁₀ monocarboxylic acids, and
         (A2b) monoethylenically unsaturated C₄-C₁₀ dicarboxylic acids, and
      (A3) optionally from 0 to 30 % by weight of other ethylenically unsaturated monomers which are copolymerizable with (A1) and (A2),
         the amounts of monomers being based in each case on the total amount of all monomers employed;
   (B) a poly(meth)acrylic binder with an average molecular mass Mn of 40,000 to 250,000, obtained by radical emulsion polymerization of at least 50 % by weight (based on the total amount of all monomers employed) of one or more monomers of formula (I), 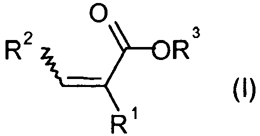 wherein
      R¹, R² and R³ are independently selected from C₁- to C₁₀-alkyl, which is optionally fluoro substituted and which is linear or branched, for example methyl, ethyl, n-propyl, i-propyl, n-butyl, i-butyl, sec-butyl, t-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl, n-decyl, preferably C₁- to C₄-alkyl, for example methyl, ethyl, n-propyl, i-propyl, n-butyl, i-butyl, sec-butyl, tert-butyl; substituted or unsubstituted aryl, preferably substituted or unsubstituted C₆- to C₁₀-aryl, more preferably substituted or unsubstituted C₆-aryl, for example phenyl or tolyl;
      R¹ and R² are optionally H;
   (C) a polyurethane binder, obtainable by reaction of the following components:
      (C1) at least one diisocyanate or polyisocyanate, preferably aliphatic, cycloaliphatic, araliphatic and/or aromatic insocyanates, more preferably diisocyanates, which are optionally biuretisized and/or isocyanurized, most preferably 1-isocyanato-3,3,5-trimethyl-5-isocyanatomethylene cyclohexane (IPDI) and hexamethylene diisocyanate-1,6 (HMDI);
      (C2) at least one diol, triol or polyol, preferably aliphatic, cycloaliphatic and/or araliphatic diols having 2 to 14, preferably 4 to 10 carbon atoms, more preferably 1,6-hexanediol or neopentyl glycol;
      (C3) optionally further components, preferably adipic acid or carbonyl diimidazole (CDI); and
      (C4) optionally further additives;
   (D) a polyisocyanurate binder comprising groups of the formula (II) 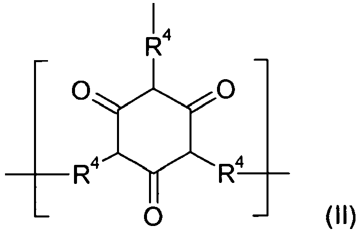 wherein R⁴ is an alkylene or arylene residue depending on the isocyanate employed in the preparation of the isocyanurate.
c) water or an aqueous solvent mixture comprising at least 65 % by weight (based on the total solvent mixture (c)) of water.

In a further aspect of the invention there is provided a method for protecting a textile material from biofouling in an aquatic environment, comprising the step of treating the textile material with the antifouling agent of the invention.

In yet a further aspect of the invention there is provided the treated textile material for application in an aquatic environment.

The treated textile material of the invention has an excellent efficacy and stability, specifically in sea water.

### Textile material

The treated material for use in aquatic, preferably subaqueous environments is a textile material. The term "textile material" as used herein includes but is not limited to fibers, yarns, wovens, nonwovens, formed-loop knits, drawn-loop knits.

In a preferred embodiment of the invention, the textile material is a fabric material and in particular a netting. The fabric material or the netting may be made of a variety of natural and synthetic fibers, also as textile blends in woven or non-woven form, as knit goods or fibers. Natural fibers are for example cotton, wool, silk, jute or hemp. Synthetic fibers may be made of polyamides, polyesters, polyacrylonitriles, polyolefines, for example polypropylene or polyethylene, Teflon, and mixtures of fibers, for example mixtures of synthetic and natural fibers. Polyamides, polyolefins and polyesters are preferred as fiber material. Polyamides and polyolefins are especially preferred.

The mesh sizes (in the case of square meshes) of the preferred nettings depend on the size of the organism that is to be caught or cultured. It is generally in the range of from 2 mm to 30 mm, preferably from 10 mm to 30 mm, in particular 15 mm to 30 mm.

Persons skilled in the art know that the mesh size of the netting should be adapted to the size of the farmed organisms during their growth. Netting may also be used to prevent predators (fish or marine mammals such as seals, sea lions, sea leopards etc.) from feeding on the farmed animals. The mesh size used in such protective netting will be adapted to the size of the predators.

Nettings according to the invention, which are treated with an antifouling agent of the invention, are generally made from textile fibres, such fibres generally having a thickness of from 0.05 mm to 10 mm. Preferably, the fibres are arranged in such a way that the net comprises a pattern of meshes with an even number of edges, where the meshes are selected from the group consisting of:
- tetragonal meshes in the shape of a rhomboid with the sides a and b and a height hₐ, where the height hₐ is from 2 mm to 30 mm, and the length to height ratio b/hₐ is 1:1 to 5:1,
- hexagonal meshes, having three pairs of parallel sides, a, b, c, with a distance of hₐ, h_{b} and h_{c} between the respective pairs of sides, where the distance hₐ is from 0.05 mm to 10 mm, and the ratio ((h_{b}+ hₐ)/2) / hₐ is 1:1 to 5:1, and
- octagonal meshes, having four pairs of parallel sides, a, b, c and d, with a distance of hₐ, h_{b}, h_{c} and h_{d} between the respective pairs of sides, where the distance hₐ is from 2 mm to 30 mm, and the ratio ((h_{b} + h_{c} + h_{d}) / 3) /hₐ is 1:1 to 5:1,
and where the terms "length" and "height" refer to the respective hole size (mesh size).

### Antifouling agent

The antifouling agent of the invention comprises an organic antifouling biocide (a), a polymeric binder (b), a solvent component (c) and optionally further components (d).

### Organic antifouling biocide (a)

The term "organic antifouling biocide" as used herein comprises any kind of active ingredient suitable for controlling organisms causing the fouling of textile materials in aquatic environment comprising at least one organic component. The term thus includes general biocides, bactericides, algicides, molluscicides, arthropodicides, fungicides, herbicides, repellents, deterrents, anti-adhesive agents (disruption of attachment), growth inhibitors, locomotion inhibitors, suppressors of metamophosis, anaesthetic and narcotizing agents, agents which block specific enzymes which are required for colonization or enzymes which destroy or impede such activity, like e.g. proteases, hydrolases, oxidases, peroxidases, superoxide permutases, catalases and the like; and/or pH-decreasing agents.

Suitable antifouling biocides are for example: chlorothalonil (tetrachloroisophthalodinitril), dichlofluanide (N,N-dimethyl-N'-phenyl-N'-(fluorodichloromethylthio) sulphamide), tolylfluanide (N,N-dimethyl-N'-tolyl-N'dichlorofluoromethylthiosulphamide), diuron (1,1-dimethyl-3-(3,4-dichlorophenyl)urea), picolinafen (N-(4-fluorophenyl)-6-[3-(trifluoromethyl)phenoxy]-2-pyridinecarboxamide, dithianon (2,3-dicyano-1,4-dithiaanthraquinone), Irgarol 1051 (N'-tert.-butyl-N-cyclopropyl-6-(methylthio)-1,3,5-triazine-2,4-diamine), 2,4,5,6-tetrachloro isophthalonitrile, 4,5-dichloro-2-octyl-3-(2H)-isothiazolin-3-one (DCOIT), 2-(thiocyanomethylthio)benzothiazole, (thiocyanomethylthio)benzothiazole, N-cyclohexyldiazeniumdioxide copper or potassium salt (CuHDO, KHDO), 2-mercapto-benzothiazole, zinc or copper pyrithione, metal dithiocarbamates, e.g. Zn, Mn, Fe or Cu ethylene *bis* (dithiocarbamate), ethylenethiuram monosulphide or disulphide, mercapto-imidazoline, diphenyl-guanidine, di-o-tolylguanidine, N,N-dialkyl dithiocarbamic acid thio anhydride, Na-N-methyl dithiocarbamate, N,N-dialkyl dithiocarbamic acid metal salts (Zn, Fe, Cd, Cr, Cu, Ni, Mn), N-acyl-1,2,4-thiazoles, N-acyl-1,3,4-thiazoles, N-acyl-1,2,3-thiazoles, N-acyl-1,2,5-thiazoles, 2-(4-thiazolyl)-benzimidazole, derivated g-lactones, benzotriazoles (e.g., 2-(2'-hydroxyphenyl) benzotriazole-5,5'-dicarboxylic acid), substituted N-aryl dichloromaleimides like e.g. N-(4-fluorophenyl)-2,3-dichloromaleimide, 2,4,6-trichlorophenyl maleimide, methyl N-(3,4-dichlorophenyl)carbamate, naphthenic acid metal salts, tetra alkyl thiuram sulphides or disulphides or their metal salts, 2,2-dithio-bis(pyridine-O-oxide), naphtha-furane derivatives according to JP09227308, triphenyl borane alkylamine complexes, e.g. triphenyl pyridine borane, (4-isopropylpyridino)methylsiphenylboron, (thiocyanoalkyl) thiobenzoheterozole, N-phenyl maleimide derivatives, N-phenyl succinic imide derivatives, fluorodichloromethyl thiophthalimides, dichloro naphthoquinone, amino chloro naphthoquinone, N-trichloromethylthio-cyclohex-4-ene-1,2-dicarboxyimide (=N-trichloromethylthio tetrahydrophthalimide) or other halogenated N-alkylthio derivatives, N-thioalkyl phthalimide derivatives such as N-(fluorodichloromethylthio)phthalimide, optionally halogenated or otherwise substituted pyridine -8-sulphonic acid esters, ethylene-bis-dithiocarbamic acid or its metal salts (salts e.g. of zinc or manganese), dimethyldithiocarbamic acid or its metal salts, 2-(thiazol-4-yl)benzimidazole, tetrachloro isophthalodinitrile, substituted coumarin derivatives, 2-thio-perhydro-1,3,5-thiadiazone derivatives, 3-(3,4-dichlorophenyl)-1,1-dimethyl urea, 3,4-dichlorocarbanilic acid methyl ester, N-propinoyl indoline, substituted N-propinoyl aniline derivatives, substituted N-thiocarbonyl indoline derivatives like e.g. 1-(benzylthiocarbonyl)indoline, dithiolo(4,5-b)quinoxalin-2-(thi)one, substituted (haloalkinyl) aryl ether such as e.g. pentachlorophenyl-iodopropargyl ether, alkyl or trifluoromethyl substituted N-phenyl benzamides, N-substituted 4-phenyl-1,3-thiazoline-2-one derivatives, 1-(4-chlorophenyl)-1-cyclopentane carboxylic acid or its amides or esters, N-substituted 5-(4-pyridyl-methyl)-6-thio-1-thia-3,5-diazacyclohexane derivatives, metal (e.g., zinc) dibenzyl dithiocarbonate, 2-methylthio-4,6-bis (isopro-pylamino)-s-triazine, 2-methyl(thio-4-tert.-butylamino-6-cyclopropylamino-s-triazine, 2-amino-3-chloro-1,4-naphthoquinone, 4-bromo-2-(4-chlorophenyl)-5-(trifluoromethyl)-1 H-pyrrole-3-carbonitrile, nostocarboline derivatives (EP 1783128), substituted 1,2-dihydroxyquinoline derivatives, e.g. 6-ethoxy-1,2-dihydroxy-2,2,4-trimethylquinoline; 1,4,2-oxathiazine derivatives such as 5,6-dihydro-3-(2-thienyl)-1,4,2-oxathiazine-4-oxide, 5,6-dihydro-3-(benzo[b]thien-2-yl)-1,4,2-oxathiazine-4-oxide, 3-(4-chlorophenyl)-5,6-dihydro-3-(2-thienyl)-1,4,2-oxathiazine4,4-dioxide, 1,2,3,4-tetrahydro-2-methyl-1,4-dioxo-2-naphthalene sulfonic acid sodium salt, 3-iodopropargyl-N-butylcarbamate (IPBC), 3-(3-iodopropargyl)-benzoxazol-2-one, 3-(3-iodopropargyl)-6-chloro-benzoxazol-2-one, and further 3-isothiazolones as disclosed in EP-A 1 142 477.

A suitable molluscicide is niclosamide. Mitochondrial electron transport inhibitors such as tebufenpyrad, pyridaben, fenazaquin are also suitable.

Possible anti-adhesives include N,N,N',N'-tetramethylethylenediamine, sulphate of p-coumaric acid (zosteric acid). Repellants: 12-epi-deoxoscalarin, α-acetoxypukalide, furospongolides, indole, phenylthiourea, tannic acid, benzoic acid, and alginic acids,

Antifouling biocides may also be natural compounds or semisynthetic actives such as gallic acid, ellagic acid and its hydrolysate, catechol, phloroglucinol; tannins, e.g. from chestnut, mimosa or quebracho trees; barettin, 8,9-dihydrobarettin; terpenes such as chloromertensine, aerothionine, homoaerothionine, other natural agents: terpenes such as palitoxin, chloromertensine, renillafoulins, pukalide, epoxypukalide, b-bisabolene; aerothionine, homoaerothionine, siphonodictidine, heteronemin, ambiol A, pallescensin A, idiadione, d-cadinen cyan, homarine, eudistomins, 2,5,6-tribromo-1-methylgramine, indole derivatives such as eudistomins or 2,5,6-tribromo-1-methylgramine and other compounds as e.g. described in N. Fusetani, Nat. Prod. Rep., 2004, 21, 94-104, or in A.I. Railkin: Marine Biofouling, CRC Press 2004.

Natural compounds which aquatic organisms (basibionts) use as a biodefence against organisms trying to foul them (epibionts) having a repelling or biocidal effect, may also be used.

The organic antifouling biocides of the invention may be used as a single active compound, in combination with other biocides or pesticides or in combination with inorganic antifoulants, especially copper and its salts such as copper sulphate, copper isothiocyanate, copper pyrithion, copper alkanoate, copper nitrate, copper thiocyanate, copper chloride, copper borate, cuprous oxide and/or zinc oxide, copper 8-quinolinolate, zinc pyrithione, zinc and/or copper fluorosilicate, copper salt of 2-pyridine-thiol-1-oxide.

A person skilled in the art will be aware that the diversity of fouling organisms may require the application of more than one active, especially the combination of actives of different modes of action. As an example, anti-locomotive protection is only efficient against those species whose dispersal forms are motile. It will obviously fail in the case of immotile spores of macroalgae and many microfoulers. In an analogy, repellants and biocides may not efficiently repel or kill all sessile foulers.

Preferred as organic antifouling biocide are CuHDO, picolinafen and dithianon.

Antifouling biocides used in the invention and their degradation products should have no or at least a low toxicity to man, other mammals, commercial aquaculture organisms like fish, mollusks such as mussels, scallops or the like, crustaceans, and macroalgae.

Suitable antifouling biocides may be selected by the skilled artisan depending on the intended use of the formulation or more specifically the intended use of the textile material to be treated with the formulation.

For use in aquaculture it is also possible to include a parasiticide together with the antifouling biocide in order to control parasites of fish, molluscs, and crustaceans, such as sea lice.

Preferred for this application are pyrethroids, like allethrin, bifenthrin, cyfluthrin, cyhalothrin, cyphenothrin, cypermethrin, alpha-cypermethrin, beta-cypermethrin, zeta-cypermethrin, deltamethrin, empenthrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, and dimefluthrin; nicotinic receptor agonists/antagonists compounds, like clothianidin, dinotefuran, imidacloprid, thiamethoxam, nitenpyram, acetamiprid, thiacloprid; the thiazol compound of formula (Γ¹) 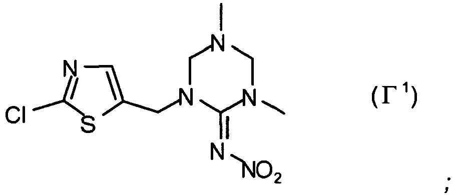 organophosphates, like dichlorvos and azamethiphos;
and macrocyclic lactone insecticides, like abamectin, emamectin, ivermectin, milbemectin, lepimectin, and spinosad.

Preferred are cypermethrin, deltamethrin, imidacloprid, ivermectin, dichlorvos, and azamethiphos.

The total amount of antifouling biocide(s) in the antifouling agent is preferably from 0 to 1 % + 50 % by weight, in particular 1 to 15 % by weight.

### Polymeric binder (b)

The biofouling agent furthermore comprises at least one polymeric binder (b) dispersed or emulgated.

In one preferred embodiment the polymeric binder is a polyethylenic binder (A), which comprises at least ethylene (A1) and an unsaturated carboxylic (A2) acid as monomers.

The polymeric binder (A) has an average molecular mass Mₙ in the range from 1500 to 20 000 g/mol, preferably 2000 to 15 000 g/mol, determinable for example by gel permeation chromatography (GPC).

As monomer (A1) the polymeric binder comprises from 60 to 95 % by weight of ethylene. Preferably, the amount of ethylene is from 70 to 80 % by weight. The amounts of monomers are being based in each case on the total amount of all monomers employed.

Furthermore, the polymeric binder (A) comprises at least one monoethylenically unsaturated carboxylic acid (A2) selected from the group of monoethylenically unsaturated C₃-C₁₀ monocarboxylic acids (A2a), and monoethylenically unsaturated C₄-C₁₀ dicarboxylic acids (A2b).

As monoethylenically unsaturated carboxylic acid (A2a) it is preferred to select at least one carboxylic acid of the general formula (III) 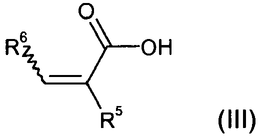 in which the variables are defined as follows:
R⁵ and R⁶ are identical or different, and are selected from hydrogen and unbranched and branched C₁-C₁₀ alkyl, such as methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl; more preferably C₁-C₄ alkyl such as methyl, ethyl, n-propyl, isopropyl, n-butyl, isobutyl, sec-butyl, and tert-butyl; and especially methyl;
R⁶ is with very particular preference hydrogen.

In one embodiment of the present invention R⁵ is hydrogen or methyl. With very particular preference R⁵ is methyl.

In one embodiment of the present invention R⁵ is hydrogen or methyl and R⁶ is hydrogen.

With very particular preference the ethylenically unsaturated carboxylic acid used of the general formula (III) is methacrylic acid.

Monomer (A2b) is at least one monoethylenically unsaturated dicarboxylic acid of the general formula

(HOOC)R⁷C=CR⁸(COOH) (IV); and/or

R⁷R⁸C=C(-(CH₂)ₙ-COOH)(COOH) (V)

R⁷ and R⁸ independently of one another are H or a straight-chain or branched, optionally substituted alkyl radical having 1 to 20 carbon atoms. Preferably the alkyl radical has 1 to 4 carbon atoms. More preferably R⁷ and/or R⁸ are/is H and/or a methyl group. The alkyl radical may also optionally contain further substituents, provided they have no adverse effect on the performance properties of the polymer or of the process.

In the case of the formula (IV) a further possibility is for R⁷ and R⁸ together to be an alkylene radical having 3 to 20 carbon atoms which may also optionally be substituted further. Preferably the ring formed from the double bond and the alkylene radical comprises 5 or 6 carbon atoms. Examples of alkylene radicals comprise in particular a 1,3-propylene or a 1,4-butylene radical, which may also contain further alkyl group substituents. n is an integer from 0 to 5, preferably 0 to 3 and very preferably 0 or 1.

It is also possible to use mixtures of two or more different monomers (A2b). In the case of (IV) the monomer in question may in each case be the cis form and/or the trans form. The monomers can also be used in the form of the corresponding carboxylic anhydrides or other hydrolyzable carboxylic acid derivatives. Where the COOH groups are located in this form it is possible with particular advantage to use cyclic anhydrides.

Examples of suitable monomers (A2b) of the formula (II) comprise maleic acid, fumaric acid, methylfumaric acid, methylmaleic acid, dimethylmaleic acid and also if appropriate the corresponding cyclic anhydrides. Examples of formula (V) comprise methylenemalonic acid and itaconic acid. Preference is given to using monomers of the formula (IV), particular preference being given to maleic acid and/or maleic anhydride.

The amount of all unsaturated carboxylic acids (A2) together is from 4 to 40 % by weight, preferably from 20 to 30 % by weight.

Besides the monomers (A1) and (A2) it is optionally possible to use one or more ethylenically unsaturated monomers (A3) copolymerizable with (A1) and (A2). Other than these no further monomers are used.

The monomers (A3) serve to fine-tune the properties of the copolymer. Of course two or more different monomers (A3) can also be used. They are selected by the skilled worker in accordance with the desired properties of the copolymer.

Examples of monomers (A3) comprise olefins, in particular α-olefines like propylene, 1-butene, 1-hexene, 1-octene, 1-decene, or 1-dodecene, styrene or other olefins like 2-butene or isobutene, furthermore one or more C₁-C₁₀ alkyl esters or α-hydroxy-C₂-C₁₀ alkylene esters of an ethylenically unsaturated C₃-C₁₀ carboxylic acid, such as methyl acrylate, ethyl acrylate, methyl methacrylate, ethyl methacrylate, n-butyl acrylate, 2-hydroxyethyl (meth)acrylate, 2-ethylhexyl (meth)acrylate or n-butyl methacrylate, for example. Further examples include monoethylenically unsaturated monomers comprising P-groups, such as vinylphosphonic acid.

The amount of additional monomers (A3) is from 0 to 30 % by weight, preferably from 0 to 9 % by weight and very preferably from 0 to 4 % by weight. Very preferably, there are no monomers (A3) present.

In general, the polymeric binder (A) has a melt flow rate (MFR) in the range from 1 to 150 g/10 min, preferably 5 to 20 g/10 min, more preferably 7 to 13 g/10 min, measured at 160°C under a load of 325 g in accordance with EN ISO 1133.

In general, the polymeric binder (A) may have an acid number in the range from 30 to 190 mg KOH/g wax, preferably 155 to 180 mg KOH/g wax, determined in accordance with DIN EN 2114.

In general, the melting range of polymeric binder (A) is in the range from 60 to 110°C, preferably in the range from 65 to 90°C, determined by DSC in accordance with DIN 51007.

In general, the density of the polymeric binder (A) is in the range from 0.89 to 0,99 g/cm³, preferably 0.89 to 0.96 g/cm³, determined in accordance with DIN 53479.

Polymeric binders (A) of ethylene (A1), ethylenically unsaturated carboxylic acids (A2) and optionally further comonomers (A3) may be prepared advantageously by free-radically initiated copolymerization under high-pressure conditions, such as in stirred high-pressure autoclaves or in high-pressure tube reactors, for example, and preferably in combinations of stirred high-pressure autoclaves and high-pressure tube reactors. Stirred high-pressure autoclaves are known per se: a description is found in Ullmann's Encyclopedia of Industrial Chemistry, 5th edition, entry heading: Waxes, Vol. A 28, pp. 146 ff., Verlag Chemie Weinheim, Basle, Cambridge, New York, Tokyo, 1996. The length/diameter ratio in such autoclaves is predominantly in ranges from 5:1 to 30:1, preferably 10:1 to 20:1. The high-pressure tube reactors which it is equally possible to employ are likewise found in Ullmann's Encyclopedia of Industrial Chemistry, 5th edition, entry heading: Waxes, Vol. A 28, pp. 146 ff., Verlag Chemie Weinheim, Basle, Cambridge, New York, Tokyo, 1996.

Suitable pressure conditions for the copolymerization are 500 to 4000 bar, preferably 1500 to 2500 bar. Conditions of this kind are also referred to below as high pressure. The reaction temperatures are in the range from 170 to 300°C, preferably in the range from 195 to 280°C.

The copolymerization can be carried out in the presence of a regulator. Suitable regulators are known to the skilled artisan. Examples have been disclosed in WO 2007/009909, page 8, line 18 to page 9, line 4.

Initiators which can be used for the free-radical polymerization are the typical free-radical initiators such as organic peroxides, oxygen or azo compounds, for example. Mixtures of two or more free-radical initiators are suitable as well. Examples have been disclosed in WO 2007/009909, page 9, line 10 to page 10, line 16.

Comonomers (A1), (A2), and optionally (A3) are typically metered together or separately. Comonomers (A1), (A2), and optionally (A3) can be compressed in a compressor to the polymerization pressure. In another embodiment of the method of the invention the comonomers are first brought by means of a pump to an increased pressure of, for example, 150 to 400 bar, preferably 200 to 300 bar, and in particular 260 bar, and then brought with a compressor to the actual polymerization pressure.

The proportion of the comonomers (A1), (A2), and optionally (A3) in the case of metered addition typically does not correspond exactly to the proportion of the units in the polymeric binder (A) since ethylenically unsaturated carboxylic acids are generally incorporated more readily into the polymeric binder (A) than is ethylene.

The copolymerization may optionally be carried out in the absence and in the presence of solvents. Examples of suitable solvents include toluene, isododecane, and isomers of xylene.

The polymeric binder (A) is used in form of its aqueous dispersion. Preferably, an aqueous dispersion of the binder (A) is prepared in a separate step and such aqueous dispersion is used for manufacturing the antifouling agent according to the invention. However, it is also possible to isolate the binder (A) as a solid after the polymerization process and to use as a solid for the antifouling agent.

The polymeric binder (A) used according to the invention may be at least partly neutralized, for example with hydroxide and/or carbonate and/or bicarbonate of alkali metal, for example, sodium hydroxide, potassium hydroxide, sodium carbonate, potassium carbonate, sodium bicarbonate, potassium bicarbonate, lithium hydroxide, or preferably with one or more amines, such as, for example, ammonia and organic amines, such as, for example, alkylamines, N-alkylethanolamines, alkanolamines and polyamines. The following may be mentioned by way of example for alkylamines: triethylamine, diethylamine, ethylamine, trimethylamine, dimethylamine, methylamine, piperidine and morpholine. Preferred amines are monoalkanolamines, N,N-dialkylalkanolamines, N-alkylalkanolamines, dialkanolamines, N-alkylalkanolamines and trialkanolamines having in each case 2 to 18 carbon atoms in the hydroxyalkyl radical and, if appropriate, in each case, 1 to 6 carbon atoms in the alkyl radical, preferably 2 to 6 carbon atoms in the alkanol radical and, if appropriate, 1 or 2 carbon atoms in the alkyl radical. Ethanolamine, diethanolamine, triethanolamine, methyldiethanolamine, n-butyldiethanolamine, N,N-dimethylethanolamine and 2-amino-2-methylpropan-1-ol are very particularly preferred. Ammonia and N,N-dimethylethanolamine are very particularly preferred. The following may be mentioned by way of example as polyamines: ethylenediamine, tetramethylethylenediamine (TMEDA), diethylenetriamine and triethylenetetramine.

Preferably, the polymeric binder (A) is partly neutralized, i.e. at least one third, more preferably at least 60 mol%, of the carboxyl groups and, for example, up to 99 mol-% of the carboxyl groups of the binder (A) are neutralized.

Furthermore, it is possible to use at least one surfactant as an auxiliary for the dispersion or emulsion of binder (A) in water. In particular, the surfactant may be anionic or preferably non-ionic.

Examples of anionic surfactants comprise alkali- or ammonium salts of C₈ to C₁₂.

In particular preferred are non-ionic surfactants including but not limited to alkoxylated C₁₀-C₃₀-alkanoles, preferably comprising from 3 to 100 moles C₂-C₄-alkylene oxides, and in particular alkoxylates oxo- or fatty alcohols. Examples of very preferred alkoxylates comprise

n-C₁₈H₃₇O-(CH₂CH₂O)₈₀-H,

n-C₁₈H₃₇O-(CH₂CH₂O)₇₀-H,

n-C₁₈H₃₇O-(CH₂CH₂O)₆₀-H,

n-C₁₈H₃₇O-(CH₂CH₂O)₅₀-H,

n-C₁₈H₃₇O-(CH₂CH₂O)₂₅-H,

n-C₁₈H₃₇O-(CH₂CH₂O)₁₂-H,

n-C₁₈H₃₇O-(CH₂CH₂O)₈₀-H,

n-C₁₆H₃₇O-(CH₂CH₂O)₇₀-H,

n-C₁₆H₃₇O-(CH₂CH₂O)₆₀-H,

n-C₁₆H₃₇O-(CH₂CH₂O)₅₀-H,

n-C₁₆H₃₇O-(CH₂CH₂O)₂₄-H,

n-C₁₆H₃₇O-(CH₂CH₂O)₁₂-H,

n-C₁₃H₂₇Oo-(CH₂CH₂O)₇₀-H,

n-C₁₃H₂₇O-(CH₂CH₂O)₆₀-H,

n-C₁₃H₂₇O-(CH₂CH₂O)₅₀-H,

n-C₁₃H₂₇O-(CH₂CH₂O)₂₅-H,

n-C₁₃H₂₇O-(CH₂CH₂O)₁₂-H,

n-C₁₂H₂₅O-(CH₂CH₂O)₁₁-H,

n-C₁₂H₂₅O-(CH₂CH₂O)₁₈-H,

n-C₁₂H₂₅O-(CH₂CH₂O)₂₅-H,

n-C₁₂H₂₅O-(CH₂CH₂O)₅₀-H,

n-C₁₂H₂₅O-(CH₂CH₂O)₈₀-H,

n-C₃₀H₆₁O-(CH₂CH₂O)₈-H,

n-C₁₀H₂₁O-(CH₂CH₂O)₉-H,

n-C₁₀H₂₁O-(CH₂CH₂O)₇-H,

n-C₁₀H₂₁O-(CH₂CH₂O)₅-H,

n-C₁₀H₂₁O-(CH₂CH₂O)₃-H,

including any mixtures thereof, wherein the indices should be understood in usual manner as average number.

Dispersions of the polymeric binder (A) may be made, by mixing the binder (A), water and optionally surfactants and/or bases at temperatures of at least 70°C;

In a further preferred embodiment the polymeric binder (b) is a poly(meth)acrylic binder (B) with an average molecular mass Mₙ of 40,000 to 250,000 obtained by radical emulsion polymerization, of at least 50 % by weight (based on the total weight of all monomers employed) of one or more monomers of formula (I) as component B1, 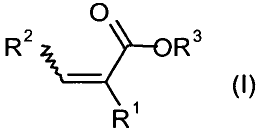 wherein
R¹, R² and R³ are independently selected from C₁- to C₁₀-alkyl, which is optionally fluoro substituted and which may be linear or branched, for example methyl, ethyl, n-propyl, i-propyl, n-butyl, i-butyl, sec-butyl, t-butyl, n-pentyl, i-pentyl, sec-pentyl, neopentyl, 1,2-dimethylpropyl, i-amyl, n-hexyl, i-hexyl, sec-hexyl, n-heptyl, n-octyl, 2-ethylhexyl, n-nonyl, n-decyl, preferably C₁- to C₄-alkyl, for example methyl, ethyl, n-propyl, i-propyl, n-butyl, i-butyl, sec-butyl, tert-butyl; substituted or unsubstituted aryl, preferably substituted or unsubstituted C₆- to C₁₀-aryl, more preferably substituted or unsubstituted C₆-aryl, for example phenyl or tolyl;
R¹ and R² may further be H.

Preferably R¹ is H or methyl. R² is preferably H. R³ is preferably methyl, ethyl, n-butyl or 2-ethylhexyl.

More preferably R¹ is H or methyl, R² is H and R³ is methyl, ethyl, n-butyl or 2-ethylhexyl.

Most preferably the monomer of formula (I) (component B1) is selected from the group consisting of 2-ethylhexylacrylate, n-butylacrylate, methylacrylate, methylmethacrylate and ethylacrylate. Most preferably a copolymer obtainable by polymerization of at least two different acrylic monomers of formula (I) is employed.

Most preferably a poly(meth)acrylic binder is used as component (b) obtained by emulsion polymerization of

B1) at least 50 % by weight (based on the total weight of all monomers employed) of one or more monomers of formula (I) as component B1, 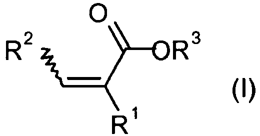 wherein
R¹, R² and R³ are independently C₁- to C₁₀-alkyl which is linear or branched, for example methyl, ethyl, n-propyl, i-propyl, n-butyl, i-butyl, sec-butyl, t-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl, n-decyl, preferably C₁- to C₄-alkyl, for example methyl, ethyl, n-propyl, i-propyl, n-butyl, i-butyl, sec-butyl, tert-butyl; substituted or unsubstituted aryl, preferably substituted or unsubstituted C₆- to C₁₀-aryl, more preferably substituted or unsubstituted C₆-aryl, for example phenyl or tolyl;
R¹ and R² are optionally H.
Preferably R¹ is H or methyl. R² is preferably H; R³ is preferably methyl, ethyl, n-butyl or 2-ethylhexyl.
More preferably R¹ is H or methyl, R² is H and R³ is methyl, ethyl, n-butyl or 2-ethylhexyl;

B2) optionally and preferably at least one monomer of formula (VI) as component B2 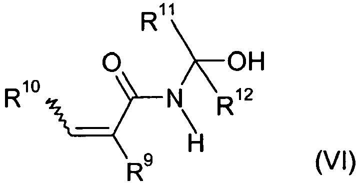 wherein
R⁹, R¹⁰, R¹¹ and R¹² are independently H, C₁- to C₁₀-alkyl which is linear or branched, for example, methyl, ethyl, n-propyl, i-propyl, n-butyl, i-butyl, sec-butyl, tert-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl and n-decyl; preferably R⁹, R¹⁰, R¹¹ and R¹² are selected from the group consisting of H, C₁- to C₄-alkyl, which may be linear or branched, for example methyl, ethyl, n-propyl, iso-propyl, n-butyl, i-butyl, sec-butyl and tert-butyl; substituted or unsubstituted aryl, preferably substituted or unsubstituted C₆-to C₁₀-aryl, more preferably substituted or unsubstituted C₆-aryl, for example phenyl or tolyl;
more preferably R⁹ is H or methyl, R¹⁰, R¹¹ and R¹² are preferably independent of each other H;
most preferably R⁹ is H or methyl and R¹⁰, R¹¹ and R¹² are H;

B3) optionally at least one monomer of formula (VII) as component B3, 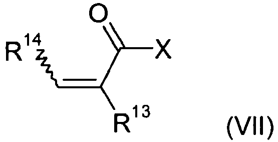 wherein
R¹³ and R¹⁴ are independently H, C₁- to C₁₀-alkyl which is linear or branched, for example, methyl, ethyl, n-propyl, i-propyl, n-butyl, i-butyl, sec-butyl, tert-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl and n-decyl; preferably R¹³ and R¹⁴ are selected from the group consisting of H, C₁- to C₄-alkyl, which is linear or branched, for example methyl, ethyl, n-propyl, iso-propyl, n-butyl, i-butyl, sec-butyl and tert-butyl; substituted or unsubstituted aryl, preferably substituted or unsubstituted C₆- to C₁₀-aryl, more preferably substituted or unsubstituted C₆-aryl, for example phenyl or tolyl;
most preferably R¹³ and R¹⁴ are H;
X is H, OH, NH₂, OR¹⁵OH, glycidyl, hydroxypropyl, 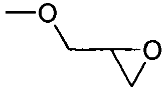 groups of the formula 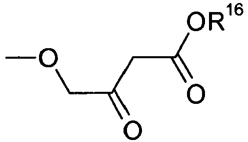 wherein
R¹⁵ is C₁- to C₁₀-alkylene, for example methylene, ethylene, propylene, butylene, pentylene, hexylene, heptylene, octylene, nonylene, decylene; preferably C₁- to C₄-alkylene, for example methylene, ethylene, propylene, butylene; substituted or unsubstituted arylene, preferably substituted or unsubstituted C₆- to C₁₀-arylene, more preferably substituted or unsubstituted C₆-arylene, for example phenylene; most preferably X ist acetoacetyl;
R¹⁶ is C₁- to C₁₀-alkyl which is branched or linear, for example methyl, ethyl, n-propyl, i-propyl, n-butyl, i-butyl, sec-butyl, tert-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl, n-decyl; preferably C₁- to C₄-alkyl, which may be branched or linear, for example methyl, ethyl, n-propyl, iso-propyl, n-butyl, iso-butyl, sec-butyl and tert-butyl; substituted or unsubstituted aryl, preferably substituted or unsubstituted C₆- to C₁₀-aryl, more preferably substituted or unsubstituted C₆-aryl, for example phenyl or tolyl;

B4) optionally further monomers which are copolymerizable with the monomers mentioned above selected from
B41) polar monomers, preferably (meth)acrylic nitrile; and/or
B42) non polar monomers, preferably styrene and/or a-methylstyrene.

The poly(meth)acrylic binder is obtained by emulsion polymerization of
B1) 50 to 100 % by weight, preferably 50 to 99 % by weight of component B1;
B2) 0 to 5 % by weight of component B2;
B3) 0 to 5 % by weight, preferably 1 to 4 % by weight, more preferably 0.2 to 3% by weight of component B3;
B4) further monomers which are copolymerizable with the monomers mentioned above selected from
   B41) 0 to 30 % by weight, preferably 0 to 25 % by weight, more preferably 5 to 20 % by weight of component B41; and/or
   B42) 0 to 40 % by weight, preferably 0 to 30 % by weight, more preferably 5 to 20 % by weight of component B42;
wherein the sum of the components B1, and optionally B2, B3 and B4 is 100 % by weight.

In a further preferred embodiment the poly(meth)acrylic binder is obtained by emulsion polymerization of
B1) 50 to 100 % by weight, preferably 50 to 99 % by weight, more preferably 50 to 95 % by weight of at least one acrylic binder (component B1) as defined above, comprising
   B11) 10 to 90% by weight, preferably 15 to 85% by weight, more preferably 30 to 85% by weight based on the acrylic binder of n-butyl acrylate;
   B12) 10 to 90% by weight, preferably 12 to 85% by weight, more preferably 15 to 65% by weight based on the acrylic binder of at least one monomer of formula (I), different from n-butyl acrylate;
B2) 0 to 5 % by weight based on the acrylic binder of at least one monomer of formula (VI) (component B2);
B3) 0 to 5 % by weight, preferably 0.1 to 4 % by weight, more preferably 0.2 to 3% by weight based on the acrylic binder of at least one monomer of formula (III) (component B3);
B4) further monomers which are copolymerizable with the monomers mentioned (component B4) above selected from
   B41) 0 to 30 % by weight, preferably 0 to 25 % by weight, more preferably 5 to 20 % by weight based on the acrylic binder of at least one polar monomer, preferably (meth)acrylic nitrile (component B1d1); and/or
   B42) 0 to 40 % by weight, preferably 0 to 30 % by weight, more preferably 5 to 20 % by weight based on the acrylic binder of at least one non polar monomer, preferably styrene and/or a-methylstyrene (component B1d2);
wherein the sum of the components B1, B2 and optionally B3 and B4 is 100 % by weight.

The poly(meth)acrylic binder may comprise further additives as known by a person skilled in the art, for example film forming agents and plasticizers, e.g. adipate, phthalate, butyl diglycol, mixtures of diesters preparable by reaction of dicarboxylic acids and alcohols which may be linear or branched. Suitable dicarboxylic acids and alcohols are known by a person skilled in the art.

Most preferably the polymeric (meth)acrylic binder is obtained by emulsion polymerization of the following components:
B1) 50 to 100 % by weight of at least one monomer of formula (I) as component B1, 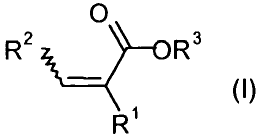 wherein
   R¹ is H or methyl, R² is H and R³ is methyl, ethyl, n-butyl, or 2-ethylhexyl, as component B1, most preferably component B1 is 2-ethylhexylacrylate, n-butylacrylate, methylacrylate, methylmethacrylate or ethylacrylate;
B2) 0 to 5 % by weight of at least one monomer of formula (VI) 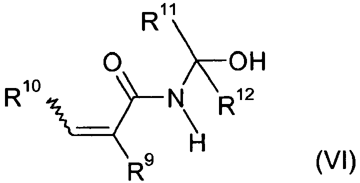 wherein R⁹ is H or methyl, R¹⁰, R¹¹ and R¹² each are H as component B2;
B3) 0 to 10 % by weight, preferably 1 to 7 % by weight, more preferably 2 to 5 % by weight of at least one monomer of formula (VII) 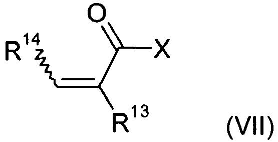 wherein R¹³ and R¹⁴ are H and X is H, OH, NH₂, OR¹⁵OH, glycidyl or a group of the formula 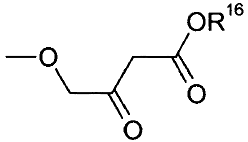 wherein
   R¹⁵ is selected from the group consisting of C₁- to C₁₀-alkylene, for example methylene, ethylene, propylene, butylene, pentylene, hexylene, heptylene, octylene, nonylene, decylene; preferably C₁- to C₄-alkylene, for example methylene, ethylene, propylene, butylenes; substituted or unsubstituted arylenes, preferably substituted or unsubstituted C₆- to C₁₀-arylene, more preferably substituted or unsubstituted C₆-arylene, for example phenylene;
   R¹⁶ is selected from the group consisting of C₁- to C₁₀-alkyl which may be branched or linear, for example methyl, ethyl, n-propyl, i-propyl, n-butyl, i-butyl, sec-butyl, tert-butyl, n-pentyl, i-pentyl, sec-pentyl, neo-pentyl, 1,2-dimethylpropyl, i-amyl, n-hexyl, i-hexyl, sec-hexyl, n-heptyl, n-octyl, 2-ethylhexyl, n-nonyl, n-decyl; preferably C₁- to C₄-alkyl, which may be branched or linear, for example methyl, ethyl, n-propyl, iso-propyl, n-butyl, iso-butyl, sec-butyl and tert-butyl; substituted or unsubstituted aryl, preferably substituted or unsubstituted C₆- to C₁₀-aryl, more preferably substituted or unsubstituted C₆-aryl, for example phenyl or tolyl;
   as component B3, most preferably X is acetoacetyl;
B4) further monomers which are copolymerizable with the monomers mentioned above selected from
   B41) 0 to 30 % by weight, preferably 0 to 25 % by weight, more preferably 5 to 20 % by weight of component B41, preferably (meth)acrylic nitrile; and/or
   B42) 0 to 40 % by weight, preferably 0 to 30 % by weight, more preferably 5 to 20 % by weight of component B42, preferably styrene and/or α-methylstyrene;
wherein the sum of components B1, B2 and optionally B3 and B4 is 100 % by weight.

In a further most preferred embodiment the amount of n-butylacrylate as component B11 is from 30 to 90 % by weight, and the other components B12 and optionally a further monomer of formula (I), component B2, B3 and B4 are chosen as mentioned before, wherein the sum of components B1, B2, B3 and B4 is 100 % by weight.

The poly(meth)acrylic binder is obtained by emulsion polymerization of the monomers mentioned before. Suitable process conditions are known by a person skilled in the art, and are disclosed e.g., in WO-A 2005/064072.

In the poly(meth)acrylic binder according to the invention, it is possible to add in general up to 10 % by weight, preferably 0.05 to 5 % by weight of mono- or di-olefinically unsaturated monomers containing reactive or cross-linking groups. Examples of such monomers are in particular the amides of α,β-olefinically unsaturated C₃-₅-carboxylic acids, particularly acryl amides, methacryl amides and maleic diamides, and their N-methylol derivatives such as N-methylol acrylic amide, N-methylol methacrylic amide, N-alkoxy methyl amides of α,β-monoolefinically unsaturated C₃₋₅-carboxylic acids such as N-methoxy methacrylic amide and N-n-butoxymethylacrylic amide, vinyl sulfonic acid, monoesters of acrylic and methacrylic acids with alkanediols such as glycol, butanediol-1,4, hexane diol-1,6, and 3-chloropropanediol-1,2, and also allyl and methallyl esters of α,β-olefinically unsaturated mono- and di-carboxylic acids such as diallyl maleate, dimethyl allyl fumarate, allyl acrylate and allyl methacrylate, diallyl phthalate, diallyl terephthalate, p-di-vinyl benzene, methylene-bis-acrylamide and ethylene glycol di-allylether.

The molecular weight of the non crosslinked emulsion polymers obtained is in general 40,000 to 250,000 (determined by GPC). The molecular weight is usually controlled by the use of conventional chain stoppers in conventional amounts. Conventional chain stoppers are for example sulfoorganic compounds.

The poly(meth)acrylic binder of the invention is obtained in form of its aqueous dispersion and is preferably employed in the biocidal agent of the present invention in form of the aqueous dispersion.

In a further preferred embodiment the polymeric binder is a polyurethane (C) obtainable by reaction of the following components:
C1) at least one diisocyanate or polyisocyanate as component C1, preferably aliphatic, cycloaliphatic, araliphatic and/or aromatic insocyanates, more preferably diisocyanates, which are optionally biuretisized and/or isocyanurized, most preferably 1-isocyanato-3,3,5-trimethyl-5-isocyanatomethylene cyclohexane (IPDI) and hexamethylene diisocyanate-1,6 (HMDI);
C2) at least one diol, triol or polyol as component C2, preferably aliphatic, cycloaliphatic and/or araliphatic diols having 2 to 14, preferably 4 to 10 carbon atoms, more preferably 1,6-hexanediol or neopentyl glycol;
C3) optionally further components as component C3, preferably adipic acid or carbonyl diimidazole (C3); and
C4) optionally further additives as component C4.

The polyurethane is preferably obtainable by reaction of the following components:
C1) 55 to 99 % by weight, preferably 70 to 98 % by weight, more preferably 75 to 90 by weight based on the polyurethane of at least one diisocyanate or polyisocyanate (component (C1), preferably aliphatic, cycloaliphatic, araliphatic and/or aromatic insocyanates, more preferably diisocyanates, which are optionally biuretisized and/or isocyanurized, more preferably alkylene diisocyanates having from 4 to 12 carbon atoms in the alkylene unit, like 1,12-dodecane diisocyanate, 2-ethyltetramethylene diisocyanate-1,4, 2-methylpentamethylene diisocyanate-1,5, tetramethylene diisocyanate-1,4, lysinester diisocyanate (LDI), hexamethylene diisocyanate-1,6 (HMDI), ciclohexane-1,3-and/or-1,4-diisocyanate, 2,4-and 2,6-hexahydro-toluylene diisocyanate as well as the corresponding isomeric mixtures 4,4'-2,2'- and 2,4'-dicyclohexylmethane diisocyanate as well as the corresponding mixtures, 1-isocyanato-3,3,5-trimethyl-5-isocyanatomethyl cyclohexane (IPDI), 2,4- and/or 2,6-toluylene diisocyanate, 4,4'-, 2,4' and/or 2,2'-diphenylmethane diisocyanate (monomeric MDI), polyphenylpolymethylene polyisocyanate (polymeric MDI) and/or mixtures comprising at least 2 of the isocyanates mentioned before; further ester-, urea-, allophanate-, carbodiimid-, uretdione- and/or urethane groups comprising di- and/or polyisocyanates may be used; most preferably 1-isocyanato-3,3,5-trimethyl-5-isocyanatomethylene cyclohexane (IPDI) and hexamethylene diisocyanate-1,6 (HMDI);
C2) 10 to 90% by weight, preferably 12 to 85% by weight, more preferably 15 to 65% by weight based on the polyurethane of at least one diol, triol or polyol (component (C2), preferably aliphatic, cycloaliphatic and/or araliphatic diols having 2 to 14, preferably 4 to 10 carbon atoms, more preferably polyols, selected from the group consisting of polyetherols, e.g. polytetrahydrofurane, polyesterols, polythioetherpolyols, hydroxyl group containing polyacetales and hydroxyl group containing aliphatic polycarbonates or mixtures of at least 2 of the polyols mentioned before. Preferred are polyesterols and/or polyetherols. The hydroxyl number of the polyhydroxy compounds is in general from 20 to 850 mg KOH/g and preferably 25 to 80 mg KOH/g. Further, diols and/or triols having a molecular weight of from in general 60 to <400, preferably from 60 to 300 g/mol are employed. Suitable diols are aliphatic, cycloaliphatic and/or araliphatic diols having from 2 to 14, preferably 4 to 10 carbon atoms, e.g. ethylene glycol, propane diol-1,3, decane diol-1,10, o-, m-, p-dihydroxycyclohexane, diethylene glycol, dipropylene glycol and preferably butane diol-1,4, neopentyl glycol, hexane diol-1,6 and bis-(2-hydroxy-ethyl)hydroquinone, triols, like 1,2,4-, 1,3,5-trihydroxycyclohexane, glycerine and trimethylol propane and mixtures of low molecular hydroxyl groups containing polyalkylene oxides based on ethylene oxide and/or 1,2-propylene oxide and the diols and/or triols mentioned before;
C3) 0 to 10 % by weight, preferably 0.1 to 5 % by weight, more preferably 1 to 5 % by weight based on the polyurethane of further components (component C3, preferably adipic acid or carbonyl diimidazole (CDI)); and
C4) 0 to 10 % by weight, preferably 0.1 to 5 % by weight, more preferably 0.5 to 5 % by weight based on the polyurethane of further additives (component C4);
wherein the sum of the components C1, C2, C3 and C4 is 100 % by weight.

The polyurethanes are prepared by methods known in the art. Further, additives as known by a person skilled in the art may be used in the process for preparing the polyurethanes.

In a further preferred embodiment the polymeric binder is a polyisocyanurate (D) comprising groups of the following formula (II): 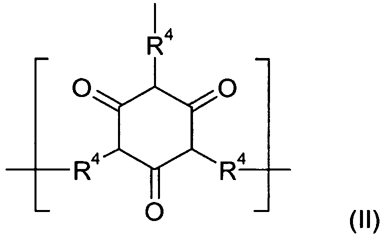 wherein R⁴ is an alkylene or arylene residue depending on the isocyanate employed in the preparation of the isocyanurate.

Polyisocyanurates are usually prepared by cyclotrimerization of isocyanates. Preferred isocyanates are the same isocyanates as mentioned before for polyurethane binder (component C1). Preparation processes and conditions for the preparation of polyisocyanurates are known by a person skilled in the art.

The polymeric binder (b) can be a mixture of two components (A), (B), (C) and (D), in particular of polyurethanes (C) and polyisocyanurates (D).

### Solvent component (c)

Preferably only water is used as solvent for the antifouling agent. However, minor amounts (e.g. less than 50 % by weight) of organic solvents miscible with water may be used in addition. Such additional solvents may be useful to improve wetting of the surfaces to be treated or to improve the solubility / dispersibility of a hydrophobic pesticide or other components in the formulation. Examples of additional solvents comprise water-miscible alcohols, e.g. monoalcohols such as methanol, ethanol or propanol, higher alcohols such as ethylene glycol or polyether polyols and ether alcohols such as butyl glycol or methoxypropanol. If an aqueous mixture is employed the mixture preferably comprises at least 65%, more preferably at least 80% and very preferably at least 95% by weight of water. The figures are based in each case on the total amount of all solvents.

### Additional components (d)

Depending on the specific binder (b) and an intended use of the textile material to be treated, the antifouling agent may further comprise one or more components selected from fixative agents, preservatives, detergents, fillers, impact modifiers, anti-fogging agents, blowing agents, clarifiers, nucleating agents, coupling agents, conductivity-enhancing agents (antistats), stabilizers such as anti-oxidants, carbon and oxygen radical scavengers and peroxide decomposing agents and the like, flame retardants, mould release agents, agents having UV protecting properties, spreading agents, antiblocking agents, anti-migrating agents, foam-forming agents, anti-soiling agents, thickeners, further biocides, wetting agents, plasticizers and film-forming agents, adhesive or anti-adhesive agents, optical brightening (fluorescent whitening) agents, pigments and dyestuffs.

Specifically in the case of acrylate binders (B), and polyurethane binders (C) the polymeric binder may advantageously be applied with a fixative agent for improved attachment of the biocide on the material. The fixative agent may comprise free isocyanate groups.

Suitable fixable agents are for example isocyanates or isocyanurates comprising free isocyanate groups. Preferably the isocyanurates are based on alkylene diisocyanates having from 4 to 12 carbon atoms in the alkylene unit, like 1,12-dodecane diisocyanate, 2-ethyltetramethylene diisocyanate-1,4, 2-methylpentamethylene diisocyanate-1,5, tetramethylene diisocyanate-1,4, lysinester diisocyanate (LDI), hexamethylene diisocyanate-1,6 (HMDI), cyclohexane-1,3-and/or-1,4-diisocyanate, 2,4-and 2,6-hexahydrotoluylene diisocyanate as well as the corresponding isomeric mixtures 4,4'-2,2'- and 2,4'-dicyclohexylmethane diisocyanate as well as the corresponding mixtures, 1-isocyanato-3,3,5-trimethyl-5-isocyanatomethyl cyclohexane (IPDI), 2,4- and/or 2,6-toluylene diisocyanate, 4,4'-, 2,4' and/or 2,2'-diphenylmethane diisocyanate (monomeric MDI), polyphenylpolymethylene polyisocyanate (polymeric MDI) and/or mixtures comprising at least 2 of the isocyanates mentioned before. More preferably the isocyanurates are based on hexamethylene diisocyanate-1,6 (HMDI).

Also preferred the isocyanurate is a isocyanurate which is hydrophilized with a polyalkylene oxide based on ethylene oxide and/or 1,2-propylene oxide, preferably polyethylene oxide.

The isocyanurate used as a fixative agent can be prepared by methods known in the art. Preferably 5 to 25% by weight, more preferably 7 to 20% by weight, most preferably 10 to 15% by weight of the isocyanate groups based on the amount of isocyanate used as staring material for the preparation of the isocyanurate are free isocyanate groups.

Preferably the isocyanurate used as a fixative agent is dissolved in a polar aprotic solvent, e.g. THF, DMF or propylene or ethylene carbonate.

A further preferred fixative agent is an isocyanurate based on HMDI which are hydrophilized with a polyethylene oxide and which is dissolved in propylene carbonate (70% by weight of HMDI in 30% by weight of propylene carbonate). The amount of free isocyanate groups is 11 to 12 % by weight, based on the amount of isocyanate used as staring material for the preparation of the isocyanurate.

If a fixative agent is used, the antifouling agent generally comprises based on the solids content of the composition 1 to 8 % by weight, preferably 1 to 5% by weight, more preferably 2 to 4 % by weight of at least one fixative agent.

As further additional compontents (d) surfactants may be used for stabilizing the antifouling biocide (a) and/or the polymeric binder (b) in the formulation. In particular preferred are anionic and/or non-ionic surfactants. Typical examples are mentioned above.

Suitable anti-foam agents are for example silicon anti-foam agents. Suitable UV-protecting agents for protecting UV-sensitive pesticides are for example paraaminobenzoic acids (PABA), octylmethoxysinameth, stilbenes, styryl or benzotriazole derivatives, benzoxazol derivatives, hydroxy-substituted benzophenones, salicylates, substituted triazines, cinnamic acid derivatives (optionally substituted by 2-cyano groups), pyrazoline derivatives, 1,1'-biphenyl-4,4'-bis-2-(methoxyphenyl)-ethenyl or other UV protecting agents. Suitable optical brighteners are dihydroquinolinone derivatives, 1,3-diaryl pyrazoline derivatives, pyrenes, naphthalic acid imides, 4,4'- diystyryl biphenylene, 4,4'- diamino-2,2'-stilbene disulphonic acids, cumarin derivatives and benzoxazole, benzisoxazole or benzimidazole systems which are linked by -CH=CH-bridges or other fluorescent whitening agents.

Typical pigments which may be used in the antifouling agent are pigments which are used in pigment dyeing or printing processes or are applied for the coloration of plastics.

Pigments may be inorganic or organic by their chemical nature. Inorganic pigments are mainly used as white pigments (e.g., titanium dioxide in the form of rutile or anatas, ZnO, chalk) or black pigments (e.g., carbon black). Colored inorganic pigments may be used as well but are not preferred because of potential toxicologic hazards. For imparting color, organic pigments or dyestuffs are preferred. Organic pigments may be mono or disazo, naphthol, benzimidazolone, (thio) indigoid, dioxazine, quinacridone, phthalocyanine, isoindolinone, perylene, perinone, metal complex or diketo pyrrolo pyrrole type pigments. Pigments may be used in powder or liquid form (i.e., as a dispersion). Preferred pigments are Pigment Yellow 83, Pigment Yellow 138, Pigment Orange 34, Pigment Red 170, Pigment Red 146, Pigment Violet 19, Pigment Violet 23, Pigment Blue 15/1, Pigment Blue 15/3, Pigment Green 7, Pigment Black 7. Other suitable pigments are known to a person skilled in the art.

Typical dyestuffs are vat dyes, cationic dyes and disperse dyes in powder or liquid form. Using the vat pigment form is preferred. Vat dyes may be of the indanthrone type, e.g. C.I. Vat Blue 4, 6 or 14; or of the flavanthrone type, e.g. C.I. Vat Yellow 1; or of the pyranthrone type, e.g. C.I. Vat Orange 2 and 9; or of the isobenzanthrone (isoviolanthrone) type, e.g. C.I. Vat Violet 1; or of the dibenzanthrone (violanthrone) type, e.g. C.I. Vat Blue 16, 19, 20 and 22, C.I. Vat Green 1, 2 and 9, C.I. Vat Black 9; or of the anthraquinone carbazole type, e.g. C.I. Vat Orange 11 and 15, C.I. Vat Brown 1, 3 and 44, C.I. Vat Green 8 and C.I. Vat Black 27; or of the benzanthrone acridone type, e.g. C.I. Vat Green 3 and 13 and C.I. Vat Black 25; or of the anthraquinone oxazole type, e.g. C.I. Vat Red 10; or of the perylene tetra carbonic acid diimide type, e.g. C.I. Vat Red 23 and 32; or imidazole derivatives, e.g. C.I. Vat Yellow 46; or amino triazine derivatives, e.g. C.I. Vat Blue 66. Other suitable vat dyes are known to a person skilled in the art. Typical disperse and cationic dyestuffs are known to the person skilled in the art.

### Preparation of the antifouling-agent

The antifouling-agent may be formed by mixing all ingredients together with water and optionally further solvents using suitable mixing and/or dispersing aggregates. In general, the formulation is formed at a temperature of from 5 to 70 °C, preferably 10 to 50 °C, more preferably 15 to 40 °C.

It is possible to use solid antifouling biocide (a), solid polymeric binder (b) and optionally additional additives (d) and to disperse them in the solvent component (c)

However, it is preferred to use dispersions of the polymeric binder (b) in water as well as formulations of the antifouling biocide (a) in water which have been separately prepared before. Such separate formulations may contain additional additives for stabilizing (a) and/or (b) in the respective formulations and are commercially available. In a second process step such raw formulations and optionally additional any additional solvents components (c) are added.

Also combinations are possible, i.e. using a pre-formed dispersion of (a) and/or (b) and mixing it with solid (a) and/or (b).

A dispersion of the polymeric binder (b) may be a pre-manufactured dispersion already made by a chemicals manufacturer.

It is possible to manufacture the antifouling agent as a final product so that it can be readily used by the end-user for the process according to the present invention.

However, it is of course also possible to manufacture a concentrate, which may be diluted by the end-user with additional solvent (c) to the desired concentration for use.

Furthermore, it may be possible to ship the formulation to the end-user as a kit comprising at least
- a first component comprising at least one antifouling biocide (a); and
- a second component comprising at least one polymeric binder (b).

Further additives (d) may be a third separate component of the kit, or may be already mixed with components (a) and/or (b).

The end-user may prepare the formulation for use by just adding water to the components of the kit and mixing.

The components of the kit may be in form of a dry composition such as a powder, a capsule, a tablet, or an effervescent tablet. Suitable pesticides in dry form are available as effervescent tablets or wettable powders which can be easily dissolved to a homogeneous formulation by manual stirring or shaking.

The components of the kit may be formulations in water or aqueous solvent mixtures. Of course it is possible to combine an aqueous formulation of one of the components with a dry formulation of the other component(s).

In a preferred embodiment of the invention the kit comprises
- one formulation of the antifouling biocide (a) and optionally solvent (c); and
- a second, separate formulation of at least one polymeric binder (b), a solvent component (c) and optionally components (d).

### Concentrations of the components

The concentration of the components (a), (b), (c) and optionally (d) will be selected by the skilled artisan depending of the technique to be used for impregnation.

In general, the amount of biocide (a) may be up to 50 % by weight, preferably 20 to 50 % based on the amount of all components (a), (b) and (d) together, i.e. all components except the aqueous solvent (c).

The amount of polymeric binder (b) may be in the range of 30 to 95 % based on the amount of all components (a), (b) and (d) together.

If present, in general the amount of additional components (d) is from 0.1 to 50 %, preferably 0.5 % to 35 % based on the amount of (a), (b), and (c) together. If present, suitable amounts of pigments and/or dyestuffs are in general 0.01 to 20 % by weight, preferably 0.1 to 10 % by weight, more preferably 0.2 to 5 % by weight, based based (a), (b), and (c).

A typical antifouling agent ready for use in impregnation processes comprises 0.01 to 25 %, preferably 0.1 to 20 % of components (a), (b), and optionally (d), the residual amount being the aqueous solvent (c).

A typical concentration of a concentrate to be diluted by the end-user may comprise 10 to 70 % of components (a), (b), and optionally (d), the residual amount being the aqueous solvent (c).

### Method of impregnation

The antifouling agent of the invention is particularly suitable for the impregnation of polyester nettings for use in fishery and aquaculture.

The method of impregnation is not limited to a specific technology of treatment. Impregnation may be performed by dipping or submerging the textile material into the formulation or by spraying the formulation onto the surface of the textile material. After treating the treated non-living material may be dried simply at ambient temperatures.

Depending on the polymeric binder (b) and the particular application it may not be necessary to perform drying at higher temperatures, crosslinking or other aftertreatment steps, though it is of course within the scope of this invention to perform such additional steps.

The formulation of the present invention may be applied to fabric materials or nettings before processing into the desired product, i.e. while still a yarn or in sheet form, or after preparation of the product.

In the case of fabrics and/or nettings, in a preferred embodiment, the invention provides a process for impregnation of fabrics and/or netting materials at least comprising the following steps:
i) treating the fabric and/or netting with the antifouling agent by any of the procedural steps selected from the group of
   (i1) passing the fabric material or netting through the antifouling agent; or
   (i2) contacting the fabric material or netting with a roller that is partly or fully dipped into the antifouling agent and drawing the antifouling agent to the side of the netting of fabric material in contact with the roller, or
   (i3) submerging the material into the antifouling agent; or
   (i4) spraying the antifouling agent onto the fabric material or netting; or
   (i5) brushing the antifouling agent onto or into the fabric material or netting; or
   (i6) applying the antifouling agent as a foam; or
   (i7) coating the antifouling agent onto fabric material or netting;
ii) optionally removing surplus formulation by squeezing the material between rollers or by means of a doctor blade; and
iii) drying and/or curing/fixing the fabric material or netting.

In case the raw materials containing residues of preceding production processes, e.g. sizes, spin finishes, other auxiliaries and/or impurities, it may be beneficial to perform a washing step before the impregnation.

Specifically, the following details are important for the steps i), ii), and iii).

### Step i1)

The antifouling agent is applied by passing the fabric or netting through the aqueous antifouling agent. Said step is known by a person skilled in the art as padding. In a preferred embodiment the fabric or netting is completely submerged in the aqueous antifouling agent either in a trough containing the liquor or the fabric or netting is passed through the antifouling agent which is held between two horizontally oriented rollers. In accordance with the invention, the fabric material or netting may either be passed through the formulation or the antifouling agent may be passed through the fabric or netting. The amount of uptake of the antifouling agent will be influenced by the stability of concentrated baths, the need for level distribution, the density of fabric or netting and the wish to save energy costs for drying and curing steps. Usual liquor-uptakes may be 40 to 150 % on the weight of material. A person skilled in the art is familiar with determining the optimum value. Step a1) is preferred for impregnating open-width material which is later tailored into nets.

Impregnation of the fabric material or netting in step a1), a2) or a3) is typically carried out at temperatures from 5 to 70 °C, preferably 10 to 50 °C, more preferably 15 to 40 °C.

### Step i3)

The netting may be impregnated by submersing it in the treatment bath. This may be done in an appropriately sized trough or vat or in a washing machine used for cleaning the nets from fouling. A person skilled in the art may choose to apply the volume of treatment bath which can be physically absorbed by the netting.

### Step i4)

The spray may be applied in continuous processes or in batch-wise processes in suitable textile machines equipped with a spraying device, e.g. in open-pocket garment washer/extractors. Such equipment is especially suitable for impregnating ready-made nets.

### Step i6)

A foam comprises less water than the solution or emulsion mentioned above. The drying process may therefore be very short. The treatment may be performed by injecting gas or blends of gas (e.g., air) into it. The addition of surfactants, preferably with film-forming properties, may be required. Suitable surfactants and the required technical equipment are known to persons skilled in the art.

### Step i7)

A coating process may preferably carried out in a doctor-blade process. The process conditions are known to a person skilled in the art.

### Step ii)

The surplus solution or emulsion is usually removed by squeezing the fabric or netting, preferably by passing the fabric material or netting through rollers as known in the art thus achieving a defined liquor uptake. The squeezed-off liquor may be re-used. Alternatively, the surplus aqueous solution or aqueous emulsion or aqueous dispersion may be removed by centrifuging or vacuum suction.

### Step iii)

An active drying process is normally performed during high scale processing. The drying is in general carried out temperatures below 200 °C. Preferred temperatures are from 50 to 170 °C, more preferably from 60 to 150 °C. The temperature choice is determined by the thermal stability of the antifouling agent and the thermal stability of the textile material impregnated. The drying can be performed in drum washing machines used for cleaning netting which are often equipped with heating. Drying may also be performed at ambient temperatures.

After or simultaneously to the drying, the impregnated textile material is optionally finally cured and/or fixated. A person skilled in the art knows how to carry out a curing and/or fixation. The curing process is in general carried out at a temperature which may be higher than the drying temperature. Preferred temperatures for curing are 60 to 170 °C, preferably 70 to 170 °C, more preferably 80 to 150 °C. Drying and curing can be advantageously be performed during one single process, e.g. in stenters with different compartments which can be heated to different temperatures. If a reactive crosslinking agent is used temperatures may be lower, e.g. 30 to 130°C, preferably 30 to 100°C.

The drying and/or curing may for example be achieved in any equipment usually applied in non-living mills for these purposes, such as stenters, loop dryers, hotflues, tumble dryers, pad steam machines etc. In one embodiment of the present invention, equipment for continuous drying and/or curing is applied. In another embodiment of the invention, equipment for discontinuous (batch-wise) drying and/or curing is used. Such equipment may comprise rotary or tumble dryers used in professional laundries, combined laundry/dryers which may be heated to the treatment temperatures, e.g. jeans stone-wash. The treatment chemicals may be added as a liquid or be sprayed onto the netting material and then brought to a homogeneous distribution by rotating the wet material before or during drying/curing. The treatment liquor may be added in excess if it is possible to remove the excess liquor e.g. by centrifuging. A person skilled in the art will be aware that treatment times might be longer than in the continuous process at the same temperature.

The curing process may also include or consist of passing the textile material by a heated surface under pressure such as an iron or a heated roller. During drying processes and curing the textile material is preferably mechanically fixated in a way to prevent change of the form e.g. shrinkage or dimensional deformation. Further, it is prevented that the biocide is washed out. The curing and/or fixation may be alternatively carried out by a dual-cure process combining heat and UV-light or only by UV-light. Suitable processes are known by a person skilled in the art.

Textiles used in aquaculture - either in freshwater or seawater (mariculture) may be in the form of netting for fish cages, netting for protecting shellfish cultures, lantern nets for pectin culture, anchor and mooring ropes or other textile equipment. The textiles used for aquaculture may be made of polyolefins, polyester, poyamide or other suitable fibers and of any colour, mesh size, and denier strength suitable for the purpose.

A typical amount of biocide (a) in the impregnated netting or fabric is from 0.1 to 25 % (dry weight) of the (dry) weight of the fabric material or netting dependent on the efficiency of the antifouling biocide. A preferred amount is between 0.5 and 20 % by weight of the fabric material or netting depending on the insecticide and/or repellent.

A typical amount of the polymeric binder (b) is from 0.1 to 25 % by weight (dry weight) of the (dry) weight of the fabric or netting. As a general guideline, the weight ratio between antifouling biocide and binder (b) should approximately be constant, i.e. the higher the amount of antifouling biocide the higher the amount of binder (b). Preferred amounts of binder (b) are from 0.5 to 20 % by weight, more preferably 1 to 20 % by weight of the (dry) weight of the fabric or netting.

The treated textile material of the invention for application in an aquatic environment preferably comprises at least one antifouling biocide selected from diuron, picolinafen, Irgarol 1051, 2,4,5,6-tetrachloro isophthalonitrile, 4,5-dichloro-2-octyl-3-(2H)-isothiazolin-3-one (DCOIT), 2-(thiocyanomethylthio)benzothiazole, (thiocyanomethylthio)benzothiazole, N-cyclohexyldiazeniumdioxide copper or potassium salt (CuHDO, KHDO), 2-mercapto-benzothiazole, zinc or copper pyrithione, metal dithiocarbamates, e.g. Zn, Mn, Fe or Cu ethylene *bis* (dithiocarbamate), ethylenethiuram monosulphide or disulphide, mercapto-imidazoline, diphenyl-guanidine, di-o-tolylguanidine, N.N-dialkyl dithiocarbamic acid thio anhydride, Na-N-methyl dithiocarbamate, N.N-dialkyl dithiocarbamic acid metal salts (Zn, Fe, Cd, Cr, Cu, Ni, Mn), N-acyl-1,2,4-thiazoles, N-acyl-1,3,4-thiazoles, N-acyl-1,2,3-thiazoles, N-acyl-1,2,5-thiazoles, 2-(4-thiazolyl)-benzimidazole, derivated g-lactones, benzotriazoles (e.g., 2-(2'-hydroxyphenyl) benzotriazole-5,5'-dicarboxylic acid), substituted N-aryl dichloromaleimides like e.g. N-(4-fluorophenyl)-2,3-dichloromaleimide, 2,4,6-trichlorophenyl maleimide, methyl N-(3,4-dichlorophenyl)carbamate, naphthenic acid metal salts, tetra alkyl thiuram sulphides or disulphides or their metal salts, 2,2-dithio-bis(pyridine-O-oxide), naphtha-furane derivatives according to JP09227308, triphenyl borane alkylamine complexes, e.g. triphenyl pyridine borane, (4-isopropylpyridino)methylsiphenylboron, (thiocyanoalkyl) thiobenzoheterozole, N-phenyl maleimide derivatives, N-phenyl succinic imide derivatives, fluorodichloromethyl thiophthalimides, dichloro naphthoquinone, amino chloro naphthoquinone, N-trichloromethylthio-cyclohex-4-ene-1,2-dicarboxyimide (=N-trichloromethylthio tetrahydrophthalimide) or other halogenated N-alkylthio derivatives, N-thioalkyl phthalimide derivatives such as N-(fluorodichloromethylthio)phthalimide, optionally halogenated or otherwise substituted pyridine -8-sulphonic acid esters, ethylene-bis-dithiocarbamic acid or its metal salts (salts e.g. of zinc or manganese), dimethyldithiocarbamic acid or its metal salts, 2-(thiazol-4-yl)benzimidazole, tetrachloro isophthalodinitrile, substituted coumarin derivatives, 2-thio-perhydro-1,3,5-thiadiazone derivatives, 3-(3.4-dichlorophenyl)-1,1-dimethyl urea, 3,4-dichlorocarbanilic acid methyl ester, N-propinoyl indoline, substituted N-propinoyl aniline derivatives, substituted N-thiocarbonyl indoline derivatives like e.g. 1-(benzylthiocarbonyl)indoline, dithiolo(4,5-b)quinoxalin-2-(thi)one, substituted (haloalkinyl) aryl ether such as e.g. pentachlorophenyl-iodopropargyl ether, alkyl or trifluoromethyl substituted N-phenyl benzamides, N-substituted 4-phenyl-1,3-thiazoline-2-one derivatives, 1-(4-chlorophenyl)-1-cyclopentane carboxylic acid or its amides or esters, N-substituted 5-(4-pyridyl-methyl)-6-thio-1-thio-3,5-diazacyclohexane derivatives, metal (e.g., zinc) dibenzyl dithiocarbonate, 2-methylthio4,6-bis (isopropylaminoys-triazine, 2-methyl(thio-4-tert.-butylamino-6-cyclopropylamino-s-triazine, 2-amino-3-chloro-1,4-naphthoquinone, 4-bromo-2-(4-chlorophenyl)-5-(trifluoromethyl)-1H-pyrrole-3-carbonitrile, nostocarboline derivatives, substituted 1,2-dihydroxyquinoline derivatives, e.g. 6-ethoxy-1,2-dihydroxy-2,2,4-trimethylquinoline; 1,4,2-oxathiazine derivatives such as 5,6-dihydro-3-(2-thienyl)-1,4,2-oxathiazine-4-oxide, 5,6-dihydro-3-(benzo[b]thien-2-yl)-1,4,2-oxathiazine-4-oxide, 3-(4-chlorophenyl)-5,6-dihydro-3-(2-thienyl)-1,4,2-oxathiazine-4,4-dioxide, 1,2,3,4-tetrahydro-2-methyl-1,4-dioxo-2-naphthalene sulfonic acid sodium salt, 3-iodopropargyl-N-butylcarbamate (IPBC), 3-(3-iodopropargylybenzoxazol-2-one, 3-(3-iodopropargyl)-6-chloro-benzoxazol-2-one, and further 3-isothiazolones as disclosed in EP-A 1 142 477.

Preferably, the polymeric binder is selected from (B), (C) or (D) and the biocide is selected from the above mentioned.

The treated textile material of the invention, which is typically in the form of a netting, is preferably used in fishery and aquaculture.

The treatment according to the invention protects the textile material form biofouling. As used herein the form "biofouling" is defined as the unwanted accumulation of microorganisms, plants and animals on materials, such as textiles, submersed into sea water or fresh water.

Biofouling is caused by various organisms including
bacteria; algae such as diatoms, red algae, brown algae, green algae, and sea weeds; animals such as protists, e.g. heterotrophic flagellates and ciliates; sponges; coelenterates, e.g. hydroids, scyphozoans, corals, and actinia (sea anemones); polychaetes; crustaceans, e.g. barnacles (cirripedes); molluscs, e.g. chitons, gastropoda, and mussels (bivalves); bryozoans; entoprocts; echinoderms, e.g. ophiurs, sea urchins, and starfishes; ascidians; and fishes.

Specific examples from the different groups include:
bacteria, e.g. achromabacter sp., alteromonas sp., a. espejina, bacillus sp., bacterium sp., caulobacter sp. , deleya (pseudomonas) marina, escherichia sp., e. coli, flavobacterium sp. , halomonas marina, hyphomicrobium sp., micrococcus sp., pseudomonas sp., p. atlantica, p. marina, p. pyocyanea, roseobacter, sarcina sp., serrratia marcescens, synechococcus sp., vibrio sp., v. campbelli, and v. vulnificus;
algae:
diatoms like achnanthes sp., amphora sp. , a. coffeaeformis, bacillaria sp., berkeleya sp., biddulphia sp., cocconeis sp., fragilaria sp., grammotophora sp., licmophora sp., melosira sp., navicula sp., nitschia sp., n. closterium, rhabdonema sp., stauroneis constricta, and synedra sp.;
red algae like ahnfeltia, a. tobuchiensis, callithamnion sp., c. corymbosum, corallina officinalis, gelidium coulteri, gigartina canaliculata, g. stellata, hildenbrandia, hydrolithon boergesenii, lithophillum, lithothamnium, nitophyllum punctatum, phycodris sp., phyllophora sp., ph. brodiae, plocamium hamatum, polysiphonia deusta, p. harveyi, and rhodymenia palmata;
brown algae like ascophyllum nodosum, chondrus crispus, cystoseira barbata, ecklonia stolonifera, ectocarpus sp., e. siliculosus, f. distichus, focus evanescens, f. inflatus, f. serratus, f. vesiculosus, laminaria sp., I. angustata var. longissima, I. hyperborean, I. japonica, I. saccharina, macrocystis pyrifera, padina sp., phyllospora comosa, sargassum tortile, and undaria pinnatifida;
green algae like chlamydomonas, cladophora rupestris, dunaliella sp., d. galbana, enteromorpha sp., e. intestinalis, e. linza, halimeda sp., u. reticulata, ulothrix sp., ulva, u. fasciata, u. lactuca, u. lobata, and urospora;
sea weeds like zostera marina
animals:
protists including heterotrophic flagellates, like bodo, codonosiga, metromonas, monosiga, pteridomonas, and spumella (monas), and
ciliates including paramecium caudatum;
sponges like aplisina fistularis, axinella sp., cliona sp., dysidea amblia, eurispongia sp., halichondria panicea, haliclona sp., h. cinerea, h. tubifera, halisarca dujardini, leoselia idia, mycale, m. cecila, ophlitaspongia seriata, phyllospongia papyracea, and siphonodictyon sp.;
coelenterates including hydroids, like clava multicornis, clava squamata, coryne uchidai, dynamena pumila, gonothyraea loveni, hydractinia echinata, laomedea flexuosa, obelia longissima, rhodymenia palmata, sertularella miurensis, tubularia, t. crocea, and t. larynx;
scyphonzoas like aurelia aurita, cassiopea andromeda, and cyanea sp. ;
corals like acropora, agaricia humilis, a. tenuifolia, galaxea aspera, leptogorgia virgulata, I. setacea, montastrea cavernosa, pachycerianthus multiplicatus, palythoa toxia, parerythropodium fulvum fulvum, renilla reniformis, sinularia sp. , s. cruciata, and xenia macrospiculata;
actinia (sea anemones) like metridium sp.;
polychaetes circeis spirillum, dexiospira brasiliensis (see neodexiospira brasiliensis), eupolymnia nebulosa, harmatoë imbricata, hydroides dianthus, h. elegans, h. norvegica, janua brasiliensis (see neodexiospira brasiliensis), mercierella enigmatica, neodexiospira (janua) brasiliensis, nereis zonata, Ophelia bicornis, pectinaria coreni, phragmatopoma, ph. californica, ph. lapidos, pigospio elegans, platinereis dumerilii, polydora sp., p. antennata, p. ciliata, pomatoceros lamarckii, protodrilus symbioticus, salmacina tribranchiata, scoloplos armiger, spirorbis sp., s. borealis, s. corallinae, s. rupestris, s. spirorbis, and s. tridentatus;
crustaceans like eupagurus;
barnacles (cirripedes), like balanus sp., b. amphitrite, b. balanus, b. cariosus, b. crenatus, b. eburneus, b. improvisus, b. perforatus, b. reticulatus, b. spongicola, chirona evermanni, chtamalus dalli, ch. stellatus, conchoderma, elminius modestus, lepas, I. hili, I. pectinata, megabalanus rosa, megabalanus tintinnabulum, pollicipes spinosus, semibalanus balanoides, solidibalanus fallax, and verruca sp.;
mollusks including chitons, like ischnochiton hakodadensis;
gastropoda, like archidoris pseudoargus, bittium reticulatum, haliotis sp., haliotis discus, h. rufescens, littorina littorea, littorina sitkana, megathura crenulata, monodonta neritoides, patella pontica, phestilla sibogae, rissoa splendida, testudinalia tessellata, tritonia plebeia, and trochus niloticus;
mussels (bivalves), like aequipecten opercularis, agropecten irradians, bankia, brachyodontes lineatus, corbicula fluminea, crassostrea gigas, c. virginica, dreissena sp., d. bugensis, d. polymorpha, geukensia demissa, haliotis rufescens, heteronomia squamula, hiatella arctica, martesia, mercenaria mercenaria, mizuhopecten yessoensis, modiolus modiolus, mytilus, m. califonianus, m. edulis, m. galloprovincialis, m. trossulus, ostrea, o. edulis, o. equestris, patinopecten yessoensis, pecten sp., p. maximus, pinna nobilis, placopecten magellanicus, saccostrea commercialis, teredo, t. navalis, t. pedicellatus, and xylophaga;
bryozoans including alcyonidium sp., a. hirsutum, a. polyoum, bowerbankia sp., b. pustulosa, bugula sp., bugula neritina, b. pacifica, b. simplex, b. stolonifera, b. turrita, callopora craticula, celleporella hyalina, conopeum sp., c. seurati, cribrillina annulata, electra sp., e. pilosa, frustrellidra hispida, membranipora sp., m. membranacea, philodophora pacifica, schizoporella unicornis, tricellaria occidentalis, watersipora cucullata, and zoobotryon pellucidum;
entoprocts including ophiurs, like ophiopholux aculeata;
sea urchins, like abracia punctulata, apostichopus japonicus, dendraster excentricus, echinarachnius parma, lytechinus pictus, paracentrotus lividius, strongylocentrotus droëbachiensis, s. intermedius, and s. purpuratus;
starfishes like asteria rubens, and pisaster giganteus;
ascidians like aplidium californicum, a. stellatum, archidistoma psammion, ascidia mentula, ascidia nigra, botryllus gigas, b. niger, b. schlosseri, bowerbankia gracilis, ciona, c. intestinalis, clavelina lepadiformis, cystodotes lobatus, dendrodoa grossularia, didemnum sp., d. candidum, diplosoma listerianum, ecteinascidia turbinata, eudistoma olivaceum, e. gladulosum, molgula citrina, m. complanata, morchellium argus, polysyncraton lacazei, piura stolonifera, styela partita, s. plicata, s. rustica, styelopsis grossularia, and trididemnum sp.; and
fishes like blennius pholis.

When used in fishery the treated textile material is preferably a net, specifically a fishing net.

The fishing net can be any type of fishing net, e.g. a trawl, a seine, a trammel, a stake net, a drift net, a gill net, a Chinese net, a cast net, a hand net, or a trap, such as a lobster trap.

Further, the treated textile material of the invention is preferably used in aquaculture (aquafarming), specifically mariculture, e.g. in fish farming, shrimp farming, prawn farming or shellfish farming, e.g. farming of mussels or oysters. The textile material is preferably used in the form of a net, e.g. a net forming a cage.

The fish include food fish, cultivated fish, aquarium fish and ornamental fish of all ages which live in fresh water, sea water and pond water. The food fish and cultivated fish include, for example, carp, eel, trout, whitefish, salmon, bream, roach, rudd, chub, sole, plaice, halibut, Japanese yellowtail (Seriola quinqueradiata), Japanese eel (Anguilla japonica), red sea bream (Pagurus major), sea bass (Dicentrarchus labrax), grey mullet (Mugilus cephalus), pompano, gilthread sea bream (Sparus auratus), Tilapia spp., chichlid species, such as, for example, plagioscion, and channel catfish.

In a specific embodiment of the invention the textile material of the invention comprises one or more pesticides for controlling parasites in fish in addition to the antifouling biocide.

Preferred pesticides have been mentioned above. The concentration of the pesticide in the textile material is generally of from 0.1 to 25 %, preferably of from 0.5 to 20 %.

Parasites of fish that can be controlled by the treated textile of the invention include Ergasilus, Bromolochus, Chondracaushus, Caligus (Caligus curtus), Lepeophtheiraus (L. salmonis), Elythrophore, Dichelestinum, Lamproglenz, Hatscheikia, Leosphilus, Symphodus, Ceudrolasus, Pseudocycmus, Lernaea, Lernaeocera, Pennella, Achthares, Basanistes, Salmincola, Brachiella, Epibrachielle, and Pseudotracheliastes, and the families Ergasilidae, Bromolochidae, Chondracanthidae, Calijidae, Dichelestiidae, Philichthyidae, Pseudocycnidae, Lernaeidae, Lernaepodidae, Sphyriidae, and Cectropidae, as well as the Branchiuriae (carp lice) with the families Argulidae and the genera Argulus spec.; as well as the Cirripediae (cirripedes; barnacles) and Ceratothoa gaudichaudii.

The invention is illustrated by the following examples without limiting it thereby.

### Examples

### Materials used

### Nettings

Two types of polyamide nets available from Egersund Rabben, Bekkjarvik, Norway, were used. All test nets had a size of 30 x 30 cm. Net (N1) had a mesh size of about 15 x 15 mm and the thread had a diameter of 2 mm. The weight of net (N1) was about 310 g/m². Net (N2) had a mesh size of about 25 x 25 mm and the thread had a diameter of about 3 mm. The weight of net (N2) was about 390 g/m².

### Organic antifouling biocide (a)

The types of organic fouling biocides were used, containing the following active ingredients:

| | |
|---|---|
| Biocide (A1): | 70 % by weight dithianon (WG-formulation), |
| Biocide (A2): | 75 % by weight picolinafen (commercial WG-formulation Sniper^{®}), |
| Biocide (A3): | 20 % by weight N-cyclohexyldiazeniumdioxide-Cu, |
| | 6.5 % by weight CuCO₃, |
| | 7.5 % by weight ethylendiamine, |
| | (66 % by weight being inert compounds). |

### Polymeric binder (b)

Three types of polymeric binders were prepared:
Binder (B1): Binder (B1) was a polyethylenic binder (A) with an average mol mass Mₙ in the range of from 1500 to 20000 g/mol based on more than 60 % by weight of ethylene.
Binder (B2): Binder (B2) was based on poly(meth)acrylates (B):

| | |
|---|---|
| 59.9 wt.-% | n-butyl acrylate, |
| 21.1 wt.-% | ethyl acrylate, |
| 18.6 wt.-% | methyl methacrylate, |
| 2.4 wt.-% | acrylic acid, |
| 0.3 wt.-% | methacrylamide. |

Binder (B3): Binder system (B3) consists of a poly(meth)acrylate binder (B) based on (% by weight):

| | |
|---|---|
| 81 % | butylacrylate, |
| 16 % | acrylnitrile, |
| 2 % | methacrylic acid-methylolamide, |
| 1 % | acrylic acid, |

and a polymeric isocyanate crosslinker based on hexamethylene diisocyanate (HMDI) (11-12 % by weight free isocyanate groups) as fixating agent.

### General Procedure

The maximum uptake of liquor was determined in pre-trials (about 60 %). The liquor (including biocide) was put in a 1 I beaker. The net sample was manually moved and kneaded until a complete take up of the liquor and a good distribution on the material had been achieved (about 5 minutes). The samples were then dried in a drying chamber with forced ventilation at 50 °C for 15 minutes.

Table 1 lists the nets according to the invention prepared according to this procedure.

**Table 1**

| **Example No.** | **Biocide Formulation g** | **Binder g** | **Weight g** | **Liquid ml** | **Net** |
|---|---|---|---|---|---|
| **1 % Biocide + 5 % Binder** | | | | | |
| 1 | 0.99 | B1 | 69.24 | 41.5 | N1 |
| | | 16.49 | | | |
| 2 | 0.99 | B2 | 69.55 | 41.7 | N1 |
| | | 7.17 | | | |
| 3 | 0.97 | B3 | 67.57 | 40.5 | N1 |
| | | 8.83 BN | | | |
| | | 0.24 FA | | | |

| **0.5 % Biocide + 5 % Binder** | | | | | |
|---|---|---|---|---|---|
| 4 | 0.50 | B1 | 69.60 | 41.8 | N1 |
| | | 16.57 | | | |
| 5 | 0.50 | B2 | 69.65 | 41.8 | N1 |
| | | 7.18 | | | |
| 6 | 0.48 | B3 | 67.75 | 40.7 | N1 |
| | | 8.85 BN | | | |
| | | 0.24 FA | | | |

| **0.5 % Biocide + 10 % Binder** | | | | | |
|---|---|---|---|---|---|
| 7 | 0.51 | B1 | 71.30 | 42.8 | N1 |
| | | 33.95 | | | |
| 8 | 0.50 | B2 | 69.58 | 41.8 | N1 |
| | | 14.35 | | | |
| 9 | 0.48 | B3 | 67.70 | 40.6 | N1 |
| | | 17.68 BN | | | |
| | | 0.47 FA | | | |

| **1 % Biocide + 5 % Binder** | | | | | |
|---|---|---|---|---|---|
| 10 | 0.93 | B1 | 69.49 | 41.7 | N1 |
| | | 16.55 | | | |
| 11 | 0.91 | B2 | 67.92 | 40.8 | N1 |
| | | 7.00 | | | |
| 12 | 0.92 | B3 | 69.30 | 41.6 | N1 |
| | | 9.05 BN | | | |
| | | 0.24 FA | | | |

| **0.5 % Biocide + 5 % Binder** | | | | | |
|---|---|---|---|---|---|
| 13 | 0.45 | B1 | 67.75 | 40.65 | N1 |
| | | 16.13 | | | |
| 14 | 0.67 | B2 | 100.85 | 60.5 | N2 |
| | | 10.40 | | | |
| 15 | 0.62 | B3 | 93.51 | 56.1 | N2 |
| | | 12.14 BN | | | |
| | | 0.33 FA | | | |

| **0.5 % Biocide + 10 % Binder** | | | | | |
|---|---|---|---|---|---|
| 16 | 0.62 | B1 | 93.47 | 56.1 | N2 |
| | | 44.51 | | | |
| 17 | 0.75 | B2 | 111.88 | 67.1 | N2 |
| | | 23.1 | | | |
| 18 | 0.63 | B3 | 94,.17 | 56.5 | N2 |
| | | 24.59 BN | | | |
| | | 0.66 FA | | | |

| **1 % Biocide + 5 % Binder** | | | | | |
|---|---|---|---|---|---|
| 19 | 4.72 | B2 9.74 | 94.43 | 56.7 | N2 |
| 20 | 4.85 | B3 | 96.93 | 58.2 | N2 |
| | | 12.65 BN | | | |
| | | 0.34 FA | | | |

| **0.5 % Biocide + 5 % Binder** | | | | | |
|---|---|---|---|---|---|
| 21 | 2.45 | B2 | 97.83 | 58.7 | N2 |
| | | 10.09 | | | |
| 22 | 2.45 | B3 | 98.12 | 58.9 | N2 |
| | | 12.81 BN | | | |
| | | 0.34 FA | | | |

| **0.5 % Biocide + 10 % Binder** | | | | | |
|---|---|---|---|---|---|
| 23 | 2.53 | B2 20.86 | 101.19 | 60.7 | N2 |
| 24 | 2.53 | B3 26.44 BN 0.71 FA | 101.26 | 60.8 | N2 |

| | | | | | |
|---|---|---|---|---|---|
| BN = Binder, FA = Fixating Agent | | | | | |

## Claims

1. The use in an aquatic environment of a textile material treated with an antifouling agent, said antifouling agent comprising
a) one or more organic antifouling biocides;
b) a polymeric binder, selected from the group consisting of
(A) a polyethylenic binder with an average molecular mass Mₙ of 1500 to 20000 g/mol, obtainable from the following monomers
(A1) from 60 to 95 % by weight of ethylene,
(A2) from 5 to 40 % by weight of at least one unsaturated carboxylic acid, selected from the group of
(A2a) monoethylenically unsaturated C₃-C₁₀ monocarboxylic acids, and
(A2b) monoethylenically unsaturated C₄-C₁₀ dicarboxylic acids, and
(A3) optionally from 0 to 30 % by weight of other ethylenically unsaturated monomers which are copolymerizable with (A1) and (A2),
the amounts of monomers being based in each case on the total amount of all monomers employed;
(B) a poly(meth)acrylic binder with an average molecular mass Mn of 40,000 to 250,000, obtained by radical emulsion polymerization of at least 50 % by weight (based on the total amount of all monomers employed) of one or more monomers of formula (I). 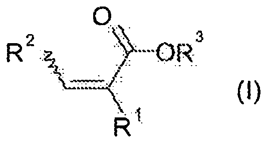 wherein
R¹, R² and R³ are independently selected from C₁- to C₁₀-alkyl, which is optionally fluoro substituted and which is linear or branched, or substituted or unsubstituted aryl, and
R¹ and R² are optionally H;
(C) a polyurethane binder, obtainable by reaction of the following components:
(C1) at least one diisocyanate or polyisocyanate;
(C2) at least one diol, triol or polyol;
(C3) optionally further components; and
(C4) optionally further additives;
(D) a polyisocyanurate binder comprising groups of the formula (II) 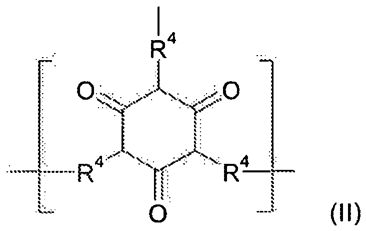 wherein R⁴ is an alkylene or arylene residue;
c) water or an aqueous solvent mixture comprising at least 65 % by weight (based on the total solvent mixture (c)) of water.

2. The use as claimed in claim 1, where the organic antifouling biocide (a) is selected from chlorothalonil (tetrachloroisophthalodinitril), dichlofluanide (N,N-dimethyl-N'-phenyl-N'-(fluorodichloromethylthio) sulphamide), tolylfluanide (N,N-dimethyl-N'-tolyl-N'dichlorofluoromethylthiosulphamide), diuron (1,1-dimethyl-3-(3,4-dichlorophenyl)urea), picolinafen (N-(4-fluorophenyl)-6-[3-(trifluoromethyl)phenoxy]-2-pyridinecarboxamide, dithianon (2,3-dicyano-1,4-dithia-anthraquinone), Irgarol 1051 (*N*'-tert.-butyl-N-cyclopropyl-6-(methylthio)-1,3,5-triazine-2,4-diamine), 2,4,5,6-tetrachloro isophthalonitrile, 4,5-dichloro-2-octyl-3-(2H)-isothiazotin-3-one (DCOIT), 2-(thiocyanomethylthio)benzothiazole, (thiocyano-methylthio)benzothiazole, N-cyclohexyldiazeniumdioxide copper or potassium salt (CuHDO, KHDO), 2-mercapto-benzothiazole, zinc or copper pyrithione, metal dithiocarbamates, e.g. Zn, Mn, Fe or Cu ethylene *bis* (dithiocarbamate), ethylenethiuram monosulphide or disulphide, mercapto-imidazoline, diphenyl-guanidine, di-o-tolylguanidine, N,N-dialkyl dithiocarbamic acid thio anhydride, Na-N-methyl dithiocarbamate, N,N-dialkyl dithiocarbamic acid metal salts (Zn, Fe, Cd, Cr, Cu, Ni, Mn), N-acyl-1,2,4-thiazoles, N-acyl-1,3,4-thiazoles, N-acyl-1,2,3-thiazoles, N-acyl-1,2,5-thiazoles, 2-(4-thiazolyl)-benzimidazole, derivated g-lactones, benzotriazoles (e.g., 2-(2'-hydroxyphenyl) benzotriazole-5,5'-dicarboxylic acid), substituted N-aryl dichloromaleimides like e.g. N-(4-fluorophenyl)-2,3-dichloromaleimide, 2,4,6-trichlorophenyl maleimide, methyl N-(3,4-dichlorophenyl)carbamate, naphthenic acid metal salts, tetra alkyl thiuram sulphides or disulphides or their metal salts, 2,2-dithio-bis(pyridine-O-oxide), triphenyl borane alkylamine complexes, e.g. triphenyl pyridine borane, (4-isopropylpyridino)methylsiphenylboron, (thiocyanoalkyl) thiobenzoheterozole, N-phenyl maleimide derivatives, N-phenyl succinic imide derivatives, fluorodichloromethyl thiophthalimides, dichloro naphthoquinone, amino chloro naphthoquinone, N-trichloromethylthio-cyclohex-4-ene-1,2-dicarboxyimide (=N-trichloromethylthio tetrahydrophthalimide) or other halogenated N-alkylthio derivatives, N-thioalkyl phthalimide derivatives such as N-{fiuorodichloromethylthio)phthalimide, optionally halogenated or otherwise substituted pyridine -8-sulphonic acid esters, ethylene-bis-dithiocarbamic acid or its metal salts (salts e.g. of zinc or manganese), dimethyldithiocarbamic acid or its metal salts, 2-(thiazol-4-yl)benzimidazole, tetrachloro isophthalodinitrile, substituted coumarin derivatives, 2-thio-perhydro-1,3,5-thiadiazone derivatives, 3-(3.4-dichlorophenyl)-1,1-dimethyl urea, 3,4-dichlorocarbanilic acid methyl ester, N-propinoyl indoline, substituted N-propinoyl aniline derivatives, substituted N-thiocarbonyl indoline derivatives like e.g. 1-(benzylthiocarbonyl)indoline, di-thiolo(4,5-b)quinoxalin-2-(thi)one, substituted (haloalkinyl) aryl ether such as e.g. pentachlorophenyl-iodopropargyl ether, alkyl or trifluoromethyl substituted N-phenyl benzamides, N-substituted 4-phenyl-1,3-thiazoline-2-one derivatives, 1-(4-chlorophenyl)-1-cyclopentane carboxylic acid or its amides or esters, N-substituted 5-(4-pyridyl-methyl)-6-thio-1-thia-3,5-diazacyclohexane derivatives, metal (e.g., zinc) dibenzyl dithiocarbonate, 2-methylthio-4,6-bis (isopropylamino)-s-triazine, 2-methyl(thio-4-tert.-butylamino-6-cyclopropylamino-s-triazine, 2-amino-3-chloro-1,4-naphthoquinone, 4-bromo-2-(4-chlorophenyl)-5-(trifluoromethyl)-1H-pyrrole-3-carbonitrile, nostocarboline derivatives substituted 1,2-dihydroxyquinoline derivatives, e.g. 6-ethoxy-1,2-dihydroxy-2,2,4-trimethylquinoline; 1,4,2-oxathiazine derivatives such as 5,6-dihydro-3-(2-thienyl)-1,4,2-oxathiazine-4-oxide, 5,6-dihydro-3-(benzo[b]thien-2-yl)-1,4,2-oxathiazine-4-oxide, 3-(4-chlorophenyl)-5,6-dihydro-3-(2-thienyl)-1,4,2-oxathiazine-4,4-dioxide, 1,2,3,4-tetrahydro-2-methyl-1,4-dioxo-2-naphthalene sulfonic acid sodium salt, 3-iodopropargyl-N-butylcarbamate (IPBC), 3-(3-iodopropargyl)-benzoxazol-2-one, 3-(3-iodopropargyl)-6-chloro-benzoxazol-2-one, and further 3-isothiazolones.

3. The use as claimed in claim 2, where the organic antifouling biocide (a) is selected from CuHDO, picolinafen and dithianon.

4. The use as claimed in any one of claims 1 to 3, where the polymeric binder (b) is a polyethylenic binder (A).

5. The use as claimed in claim 4, where the polymeric binder (A) comprises at least one monoethylenically unsaturated carboxylic acid (A2) selected from the group of monoethylenically unsaturated C₃-C₁₀ monocarboxylic acids (A2a), and monoethylenically unsaturated C₄-C₁₀ dicarboxylic acids (A2b).

6. The use as claimed in any one of claims 1 to 3, where the polymeric binder (b) is a poly(meth)acrylic binder (B).

7. The use as claimed in claim 6, where the poly(meth)acrylic binder (B) is obtained by emulsion polymerization of
B1) at least 50 % by weight (based on the total weight of all monomers employed) of one or more monomers of formula (I) as component B1, 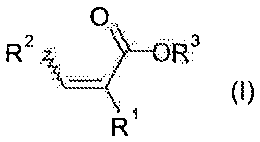 wherein
R¹, R² and R³ are independently C₁- to C₁₀-alkyl which is linear or branched, or substituted or unsubstituted aryl;
R¹ and R² are optionally H.
B2) optionally at least one monomer of formula (VI) as component B2 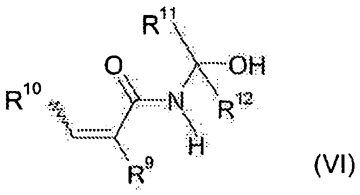 wherein
R⁹, R¹⁰, R¹¹ and R¹² are independently H, C₁- to C₁₀-alkyl which is linear or branched, or substituted or unsubstituted aryl;
B3) optionally at least one monomer of formula (VII) as component B3, 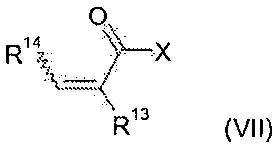 wherein
R¹³ and R¹⁴ are independently H, C₁- to C₁₀-alkyl which is linear or branched, or substituted or unsubstituted aryl;
X is H, OH, NH₂, OR¹⁵OH, glycidyl, hydroxypropyl, 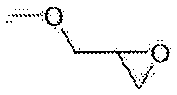 groups of the formula 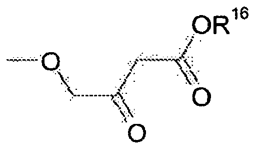 wherein
R¹⁵ is C₁- to C₁₀-alkylene, or substituted or unsubstituted arylene,
R¹⁶ is C₁- to C₁₀-alkyl which is branched or linear, or substituted or unsubstituted aryl, and
B4) optionally further monomers which are copolymerizable with the monomers mentioned above selected from
B41) polar monomers, preferably (meth)acrylic nitrile and/or methyl(meth)acrylate; and/or
B42) non polar monomers, preferably styrene and/or α-methylstyrene.

8. The use as claimed in any one of claims 1 to 4, 6 or 7, where the antifouling agent comprises a fixating agent (d).

9. The use as claimed in any one of claims 1 to 8, where the treated textile material is in the form of a net.

10. The use as claimed in claim 9, where the net has a mesh size of from 2 mm to 30 mm.

11. The use as claimed in claim 9 or 10, where the meshes have a tetragonal, hexagonal or octagonal form.

12. The use as claimed in any one of claims 1 to 11, where the treated textile material comprises, in addition to the biocide (a), a parasiticide selected from pyrethroids, nicotinic receptor agonist/antagonist compounds, organo phosphates and macrocyclic lactone insecticides.

13. The use as claimed in claim 12 in aquaculture to protect the cultured animals from parasites.

## Patentansprüche

1. Verwendung eines mit einem Antifouling-Mittel behandelten Textilmaterial in einer aquatischen Umgebung, wobei das Antifouling-Mittel Folgendes umfasst:
a) ein oder mehrere organische Antifouling-Biozide;
b) ein polymeres Bindemittel aus der Gruppe bestehend aus
(A) einem Polyethylen-Bindemittel mit einer mittlerem Molmase Mₙ von 1500 bis 20.000 g/mol, das aus den folgenden Monomeren erhältlich ist:
(A1) 60 bis 95 Gew.-% Ethylen,
(A2) 5 bis 40 Gew.-% mindestens einer ungesättigen Carbonsäure aus der Gruppe der
(A2a) monoethylenisch ungesättigten C₃-C₁₀-Monocarbonsäuren und
(A2b) monoethylenisch ungesättigten C₄-C₁₀-Dicarbonsäuren und
(A3) gegebenenfalls 0 bis 30 Gew.-% anderer ethylenisch ungesättigter Monomere, die mit (A1) und (A2) copolymerisierbar sind,
wobei sich die Monomerenmengen jeweils auf die Gesamtmenge aller eingesetzten Monomere beziehen;
(B) einem Poly(meth)acryl-Bindemittel kit einer mittleren Molmase Mₙ von 40.000 bis 250.000, das durch radikalische Emulsionspolymerisation von mindestens 50 Gew.-% (bezogen auf die Gesamtmenge aller eingesetzten Monomere) eines oder mehrerer Monomere der Formel (I) 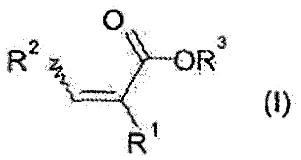 worin
R¹, R² und R³ unabhängig voneinander unter C₁- bis C₁₀-Alkyl, das gegebenenfalls fluorsubstituiert und linear oder verzweigt ist,
oder substituiertem oder unsubstituiertem Aryl ausgewählt sind und
R₁ und R₂ gegebenenfalls für H stehen, erhältlich ist;
(C) einem Polyurethan-Bindemittel, das durch Umsetzung der folgenden Komponenten erhältlich ist:
(C1) mindestens eines Diisocyanat oder Polyisocyanats;
(C2) mindestens eines Diols, Triols oder Polyols;
(C3) gegebenenfalls weiteren Komponenten und
(C4) gegebenenfalls weiteren Additiven;
(D) einem Polyisocyanurat-Bindemittel, der Gruppen der Formel (II) 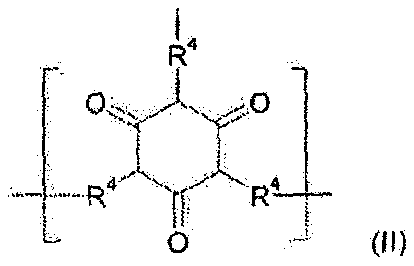 worin R⁴ für einen Alkylen- oder Arylenrest steht,
umfasst;
c) Wasser oder eine wässerige Lösungsmittenmischung, die mindestens 65 Gew.-% (bezogen auf die gesamte Lösungsmittelmischung (c)) Wasser umfasst.

2. Verwendung nach Anspruch 1, wobei das organische Antiflouling-Biozid (a) unter Chlorothalonil (Tetrachlorisophthalodinitril), Dichlofluanid (N,N-Dimethyl-N'-phenyl-N'- fluordichlormethyl-thio)sulfamid), Tolylfluanid (N,N-Dimethyl-N'-tolyl-N'-dichlorfluormethylthiosulfamid), Diuron (1,1-Dimethyl-3-(3,4-dichlorphenyl)harnstoff), Picolinafen (N-(4-Fluorphenyl)-6-[3-(trifluormethyl phenoxy -2-pyridincarboxamid, Dithianon (2,3-Dicyano-1,4-dithiaanthrachinon), Irgarol 1051 (N'-tert.-Butyl-N-cyclopropyl-6-(methylthio)-1,3,5-triazin-2,4-diamin), 2,4,5,6-Tetrachlorisophthalonitril, 4,5-Dichlor-2-octyl-3-(2H)-isothiazolin-3-on (DCOIT), 2-Thiocyanomethylthio - benzotriazol, (Thiocyano-methylthio)benzothiazol, N-Cyclohexyldiazeniumdioxid-Kupfersalz oder -kaliumsalz (CuHDO, KHDO), 2-Mercaptobenzothiazol, Zink- oder Kupferpyrithion, Metalldithiocarbamaten, z.B. Zn-, Mn-, Fe- oder Cu-Ethylenbis(dithiocarbamat), Ethylenthiurammonosulfid oder -disulfid, Mercaptoimidazolin, Diphenylguanidin, Di-o-tolylguanidin, N,N-Dialkyl-dithiocarbamidsäurethioanhydrid, Na-N-Methyldithiocarbamat, N,N-Dialkyldithiocarbamidsäure-Metallsalzen (Zn, Fe, Cd, Cr, Cu, Ni, Mn), N-Acyl-1,2,4-thiazolen, N-Acyl-1,3,4-thiazolen, N-Acyl-1,2,3-thiazolen, N-Acyl-1,2,5-thiazolen, 2-(4-Thiazolyl)benzimidazol, derivatisierten g-Lactonen, Benzotriazolen (z.B. 2-(2'-Hydroxyphenyl)benzotriazol-5,5'-dicarbonsäure), substituierten N-Aryldichlormaleinimiden wie z.B. N-(4-Fluorphenyl -2,3-dichlormaleinimid, 2,4,6-Trichlorphenylmaleinimid, N-(3,4-Dichlorphenyl - carbamidsäuremethylester, Naphthensäure-Metallsalzen, Tetraalkylthiuramsulfiden oder -disulfiden oder Metallsalzen davon, 2,2-Dithio-bis(pyridin-O-oxid), Triphenylboran-Alkylamin-Komplexen, z.B. Triphenyl-pyridin-boran, (4-Iso-propylpyridino)methyldiphenylbor, (Thiocyanoalkyl)thiobenzoheterozol, N-Phenylmaleinimid-Derivaten, N-Phenylbernsteinsäureimid-Derivaten, Fluordichlormethylthiophthalimiden, Dichlornaphthochinon, Aminochlornaphthochinon, N-Tri-chlormethylthiocyclohex-4-en-1,2-dicarboximid (= N-Trichlormethylthiotetrahydrophthalimid) oder anderen halogenierten N-Alkylthio-Derivativen, N-Thioalkylphthalimid-Derivaten wie N-(Fluordichlor-methylthio)phthalimid, gegebenenfalls halogenierten oder anderweitig substituierten Pyridin-8-sulfonsäureestern, Ethylen-bis-dithiocarbamidsäure oder Metallsalzen davon (z.B. Zink- oder Mangansalzen), Dimethyldithiocarbamidsäure oder Metallsalzen davon, 2-(Thiazol-4-yl)benzimidazol, Tetrachlorisophthalodinitril, substituierten Cumarinderivaten, 2-Thioperhydro-1,3,5-thiadiazon-Derivaten, 3-(3,4-Dichlorphenyl)-1,1-dimethylharnstoff, 3 4-Dichlorcarbanilsäuremethylester, N-Propinoylindoline, substituierten N-Propinoylanilin-Derivaten, substituierten N-Thiocarbonylindolin-Derivaten wie z.B. 1-(Benzylthiocarbonyl)indolin, Dithiolo(4,5-b)chinoxalin-2-(thi)on, substituierten (Halogenalkinyl)arylethern wie z.B. Pentachlorphenyl-iodpropargylether, alkyl- oder trifluormethylsubstituierten N-Phenylbenzamiden, N-substituierten 4-Phenyl-1,3-thiazolin-2-on-Derivates, 1-(4-Chlorphenyl)-1-cyclopentancarbonsäure oder Amiden oder Estern davon, N-substituierten 5-(4-Pyridylmethyl)-6-thio-1-thia-3,5-diazacyclohexan-Derivaten, Metalldibenzyldithiocarbonat (z.B. Zinkdibenzyldithiocarbonat), 2-Methylthio-4,6-bis(isopropylamino)-s-triazin, 2-Methy (thio-4-tert.-butylamino-6-cyclopropylamino-s-triazin, 2-Amino-3-chlor-1,4-naphthochinon, 4-Brom-2-(4-chlorphenyl)-5-trifluormethyl)-1H-pyrrol-3-carbonitril, Nostocarbolin-Derivaten, substituierten 1,2-Dihydroxychinolin-Derivaten, z.B. 6-Ethoxy-1,2-dihydroxy-2,2,4-trimethylchinolin; 1,4,2-Oxathiazin-Derivaten wie 5,6-Di-hydro-3-(2-thienyl)-1,4,2-oxathiazin-4-oxid, 5,6-Dihydro-3-(benzo[b]thien-2-yl)-1,4,2-oxathiazin-4-oxid, 3-(4-Chlorphenyl)-5,6-dihydro-3-(2-thienyl - 1,4,2-oxathiazin-4,4-dioxid, 1,2,3,4-Tetrahydro-2-methyl-1,4-dioxo-2-naphthalinsulfonsäure-Natriumsalz, 3-Iodpropargyl-N-butylcarbamat (IPBC), 3-(3-Iodpropargyl)-benzoxazol-2-on, 3-(3-Iodpropargyl) - 6-chlorbenzoxazol-2-on und weiteren 3-Isotriazolonen ausgewählt ist.

3. Verwendung nach Anspruch 2, wobei das organische Antifouling-Biozid (a) unter CuHDO, Picolinafen und Dithianon ausgewählt ist.

4. Verwendung nach einem der Ansprüche 1 bis 3, wobei es sich bei dem polymeren Bindemittel (b) um ein Polyethylen-Bindemittel (A) handelt.

5. Verwendung nach Anspruch 4, wobei das polymere Bindemittel (A) mindestens eine monoethylenisch ungesättigte Carbonsäure (A2) aus der Gruppe der monoethylenisch ungesättigten C₃-C₁₀-Monocarbonsäuren (A2a) und monoethylenisch ungesättigten C₄-C₁₀-Dicarbonsäuren (A2b) umfasst.

6. Verwendung nach einem der Ansprüche 1 bis 3, wobei es sich bei dem polymeren Bindemittel (b) um ein Polymethacryl-Bindemittel (B) handelt.

7. Verwendung nach Anspruch 6, wobei das Poly(meth)-acryl-Bindemittel (B) durch Emulsionspolymerisation von
B1) mindestens 50 Gew.-% (bezogen auf die Gesamtmenge aller eingesetzten Monomere) eines oder mehrerer Monomere der Formel (I) als Komponente B1, 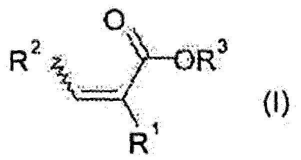 worin
R¹, R² und R³ unabhangig voreinander für C₁- bis C₁₀-Alkyl, das linear oder verzweigt ist, oder substituiertes oder unsubstituiertes Aryl stehen und
R₁ und R₂ gegebenenfalls für H stehen;
B2) gegebenenfalls mindestens einem Monomer der Formel (VI) als Komponente B2 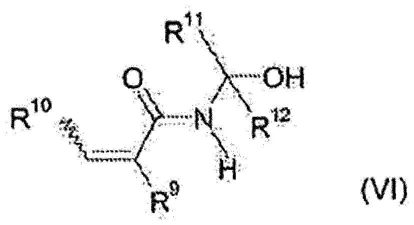 worin
R⁹, R¹⁰, R¹¹ und R¹² unabhängig voneinander für H, C₁- bis C₁₀-Alkyl, das linear oder verzweigt ist, oder substituiertes oder unsubstituiertes Aryl stehen;
B3) gegebenenfalls mindestens einem Monomer der Formel (VII) als Komponente B3 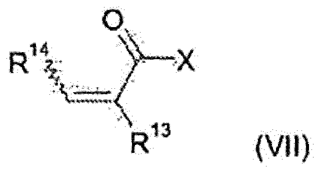 worin
R¹³ und R¹⁴ unabhangig voneinander für H, C₁- bis C₁₀-Alkyl, das linear oder verzweigt ist, oder substituiertes oder unsubstituiertes Aryl stehen und
X für H, OH, NH₂, OR¹⁵OH, Glycidyl, Hydroxypropyl, 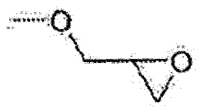 Gruppen der Formel 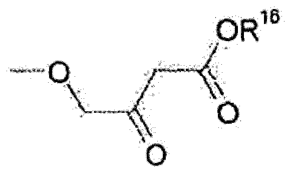 worin
R¹⁵ für C₁- bis C₁₀-Alkylen oder substituiertes oder unsubstituiertes Arylen steht,
R¹⁶ für C₁- bis C₁₀-Alkyl, das linear oder verzweigt ist, oder substituiertes oder unsubstituiertes Aryl steht,
steht; und
B4) gegebenenfalls weiteren Monomeren, die mit den oben genannten Monomeren copolymerisierbar sind, ausgewählt unter
B41) polaren Monomeren, vorzugsweise (Meth)acrylnitril und/oder Methyl(meth)acrylat; und/oder
B42) unpolaren Monomeren, vorzugsweise Styrol und/oder a-Methylstyrol;
erhalten wird.

8. Verwendung nach einem der Ansprüche 1 bis 4, 6 oder 7, wobei das Antifouling-Mittel ein Fixiermittel (d) umfasst.

9. Verwendung nach einem der Ansprüche 1 bis 8, wobei das behandelte Textilmaterial in Form eines Netzes vorliegt.

10. Verwendung nach Anspruch 9, wobei das Netz eine Maschenweite von 2 mm bis 30 mm aufweist.

11. Verwendung nach Anspruch 9 oder 10, wobei die Netze eine tetragonale, hexagonale oder oktagonale Form ausweisen.

12. Verwendung nach einem der Ansprüche 1 bis 11, wobei das behandelte Textilmaterial neben dem Biozid (a) ein Parasitizid, das unter Pyrethroiden, Nicotinrezeptoragonisten/-antagonisten, Organophosphaten und makrocyclischen Lacton-Insektiziden ausgewählt ist, umfasst.

13. Verwendung nach Anspruch 12 in der Aquakultur zum Schutz der gezüchteten Tiere vor Parasiten.

## Revendications

1. Utilisation dans un environnement aquatique d'un matériau textile traité par un agent anti-salissures, ledit agent anti-salissures comprenant :
a) un ou plusieurs biocides anti-salissures organiques ;
b) un liant polymère, choisi dans le groupe constitué par
(A) un liant polyéthylénique ayant une masse moléculaire moyenne Mₙ allant de 1500 à 20 000 g/mol, pouvant être obtenu à partir des monomères suivants
(A1) de 60 à 95% en poids d'éthylène,
(A2) de 5 à 40% en poids d'au moins un acide carboxylique insaturé, choisi dans le groupe constitué par
(A2a) les acides monocarboxyliques en C₃-C₁₀ monoéthyléniquement insaturés, et
(A2b) les acides dicarboxyliques en C₄-C₁₀ monoéthyléniquement insaturés, et
(A3) éventuellement de 0 à 30% en poids d'autres monomères éthyléniquement insaturés qui sont copolymérisables avec (A1) et (A2),
les quantités de monomères étant basées dans chaque cas sur la quantité totale de tous les monomères employés ;
(B) un liant poly(méth)acrylique ayant une masse moléculaire moyenne Mₙ allant de 40 000 à 250 000, obtenu par polymérisation radicalaire en émulsion d'au moins 50% en poids (sur la base de la quantité totale de tous les monomères employés) d'un ou plusieurs monomères de formule (I), 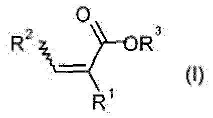 dans laquelle
R¹, R² et R³ sont choisis indépendamment parmi C₁- à C₁₀-alkyle, qui est éventuellement substitué par fluoro et qui est linéaire ou ramifié, ou aryle substitué ou non substitué, et
R¹ et R² sont éventuellement H ;
(C) un liant à base de polyuréthane, pouvant être obtenu par la réaction des composants suivants :
(C1) au moins un diisocyanate ou polyisocyanate ;
(C2) au moins un diol, triol ou polyol ;
(C3) éventuellement d'autres composants ; et
(C4) éventuellement d'autres additifs ;
(D) un liant à base de polyisocyanurate comprenant des groupements de formule (II) 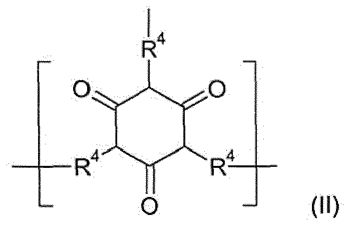 dans laquelle
R⁴ est un reste alkylène ou arylène ;
c) de l'eau ou un mélange de solvants aqueux comprenant au moins 65% en poids (sur la base du mélange total de solvants (c)) d'eau.

2. Utilisation selon la revendication 1, dans laquelle le biocide anti-salissures organique (a) est choisi parmi le chlorothalonil (tétrachloroisophtalodinitrile), le dichlofluanid (N,N-diméthyl-N'-phényl-N'-(fluorodichlorométhylthio)sulfamide), le tolylfluanide (N,N-diméthyl-N'-tolyl-N'-dichlorofluorométhylthiosulfamide), le diuron (1,1-diméthyl-3-(3,4-dichlorophényl)urée), le picolinafen (N-(4-fluorophényl)-6-[3-(trifluorométhyl)phénoxy]-2-pyridinecarboxamide, le dithianon (2,3-dicyano-1,4-dithia-anthraquinone), l'Irgarol 1051 (N'-tertio-butyl-N-cyclopropyl-6-(méthylthio)-1,3,5-triazine-2,4-diamine), le 2,4,5,6-tétrachloro-isophtalonitrile, la 4,5-dichloro-2-octyl-3-(2H)-isothiazolin-3-one (DCOIT), le 2-(thiocyanométhylthio)benzothiazole, le (thiocyanométhylthio)benzothiazole, le sel de cuivre ou de potassium du bioxyde de N-cyclohexyldiazénium (CuHDO, KHDO), le 2-mercapto-benzothiazole, la pyrithione de zinc ou de cuivre, les dithiocarbamates de métal, par exemple l'éthylène bis(dithiocarbamate) de Zn, Mn, Fe ou Cu, le monosulfure ou disulfure d'éthylène thiuram, la mercapto-imidazoline, la diphényl-guanidine, la di-o-tolylguanidine, le thio-anhydride de l'acide N,N-dialkyl-dithiocarbamique, le dithiocarbamate de Na-N-méthyle, les sels métalliques de l'acide N,N-dialkyl-dithiocarbamique (Zn, Fe, Cd, Cr, Cu, Ni, Mn), les N-acyl-1,2,4-thiazoles, les N-acyl-1,3,4-thiazoles, les N-acyl-1,2,3-thiazoles, les N-acyl-1,2,5-thiazoles, le 2-(4-thiazolyl)-benzimidazole, les g-lactones dérivées, les benzotriazoles (par exemple, l'acide 2-(2'-hydroxyphényl)benzotriazole-5,5'-dicarboxylique), les N-aryl-dichloromaléimides substitués tels que par exemple le N-(4-fluorophényl)-2,3-dichloromaléimide, le 2,4,6-trichlorophénylmaléimide, le N-(3,4-dichlorophényl)carbamate de méthyle, les sels métalliques de l'acide naphténique, les sulfures ou bisulfures de tétraalkylthiuram ou leurs sels métalliques, le 2,2-dithio-bis (pyridine-O-oxyde), les complexes d'alkylamine-triphénylborane, par exemple le pyridine-triphénylborane, le (4-isopropyl,-pyridino)méthylsiphénylbore, le (thiocyanoalkyl)-thiobenzohétérozole, les dérivés de N-phénylmaléimide, les dérivés d'acide N-phénylsuccinique imide, les fluorodichlorométhyle thiophtalimides, la dichloronaphtoquinone, l'aminochloro-naphtoquinone, le N-trichlorométhylthio-cyclohex-4-ène-1,2-dicarboxyimide (= N-trichlorométhylthiotétrahydrophtalimide) ou d'autres dérivés N-alkylthio halogénés, des dérivés de N-thioalkylphtalimide tels que le N-(fluorodichlorométhylthio)phtalimide, éventuellement halogéné ou par ailleurs substitué par des esters d'acide pyridine-8-sulfonique, l'acide éthylène-bis-dithiocarbamique ou ses sels métalliques (par exemple les sels de zinc ou de manganèse), l'acide diméthyldithiocarbamique ou ses sels métalliques, le 2-(thiazol-4-yl)benzimidazole, le tétrachloro-isophtalodinitrile, les dérivés de coumarine substitués, les dérivés de 2-thio-perhydro-1,3,5-thiadiazone, la 3-(3,4-dichlorophényl)-1,1-diméthylurée, l'acide 3,4-dichlorocarbanilique méthylester, la N-propinoylindoline, les dérivés de N-propinoylaniline substitués, les dérivés de N-thiocarbonylindoline substitués tels que exemple la 1-(benzylthiocarbonyl)indoline, la dithiolo(4,5-b)quinoxalin-2-(thi)one, les (halogénoalcynyl) aryle éthers substitués tels que par exemple le pentachlorophényl-iodopropargyle éther, les N-phénylbenzamides substitués par alkyle ou trifluorométhyle, les dérivés de 4-phényl-1,3-thiazoline-2-one N-substitués, l'acide 1-(4-chlorophényl)-1-cyclopentanecarboxylique ou ses amides ou esters, les dérivés de 5-(4-pyridylméthyl)-6-thio-1-thia-3,5-diazacyclohexane N-substitués, le dibenzyldithiocarbonate de métal (par exemple, de zinc), la 2-méthylthio-4,6-bis(isopropylamino)-s-triazine, la 2-méthyl(thio-4-tertio-butylamino-6-cyclopropylamino-s-triazine, la 2-amino-3-chloro-1,4-naphtoquinone, le 4-bromo-2- 4-chlorophényl -5-(trifluorométhyl-1H-pyrrole-3-carbonitrile, les dérivés de nostocarboline, les dérivés de 1,2-dihydroxyquinoléine substitués, par exemple la 6-éthoxy-1,2-dihydroxy-2,2,4-triméthylquinoléine ; les dérivés de 1,4,2-oxathiazine tels que le 4-oxyde de 5,6-dihydro-3-(2-thiényl)-1,4,2-oxathiazine, le 4-oxyde de 5,6-dihydro-3-(benzo[b]thién-2-yl)-1,4,2-oxathiazine, le 4,4-dioxyde de 3-4-chlorophényl-5,6-dihydro-3-(2-thiényl)-1,4,2-oxathiazine, le sel de sodium de l'acide 1,2,3,4-tétrahydro-2-méthyl-1,4-dioxo-2-naphtalènesulfonique, le 3-iodopropargyl-N-butylcarbamate (IPBC), la 3-(3-iodopropargyl)-benzoxazol-2-one, la 3-(3-iodopropargyl)-6-chloro-benzoxazol-2-one, et d'autres 3-isothiazolones.

3. Utilisation selon la revendication 2, dans laquelle le biocide anti-salissures organique (a) est choisi parmi le CuHDO, le picolinafen et le dithianon.

4. Utilisation selon l'une quelconque des revendications 1 à 3, dans laquelle le liant polymère (b) est un liant polyéthylénique (A).

5. Utilisation selon la revendication 4, dans laquelle le liant polymère (A) comprend au moins un acide carboxylique monoéthyléniquement insaturé (A2) choisi dans le groupe constitué par les acides monocarboxyliques en C₃-C₁₀ monoéthyléniquement insaturés (A2a), et les acides dicarboxyliques en C₄-C₁₀ monoéthyléniquement insaturés (A2b).

6. Utilisation selon l'une quelconque des revendications 1 à 3, dans laquelle le liant polymère (b) est un liant poly(méth)acrylique (B).

7. Utilisation selon la revendication 6, dans laquelle le liant poly(méth)acrylique (B) est obtenu par polymérisation en émulsion
B1) d'au moins 50% en poids (sur la base du poids total de tous les monomères employés) d'un ou plusieurs monomères de formule (I) comme composant B1, 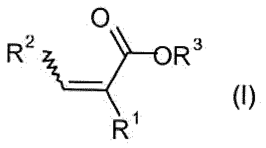 dans laquelle
R¹, R² et R³ sont indépendamment C₁- à C₁₀-alkyle qui est linéaire ou ramifié, ou aryle substitué ou non substitué ;
R¹ et R² sont éventuellement H ;
B2) éventuellement d'au moins un monomère de formule (VI) comme composant B2 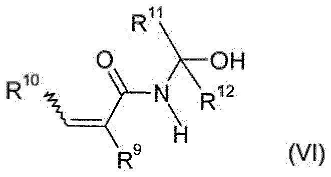 dans laquelle
R⁹, R¹⁰, R¹¹ et R¹² sont indépendamment H, C₁- à C₁₀-alkyle qui est linéaire ou ramifié, ou aryle substitué ou non substitué ;
B3) éventuellement d'au moins un monomère de formule (VII) comme composant B3, 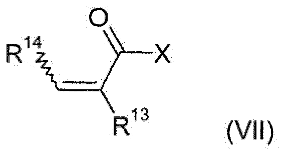 dans laquelle
R¹³ et R¹⁴ sont indépendamment H, C₁- à C₁₀-alkyle qui est linéaire ou ramifié, ou aryle substitué ou non substitué ;
X est H, OH, NH₂, OR¹⁵OH, glycidyle, hydroxypropyle, 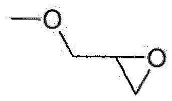 des groupements de formule 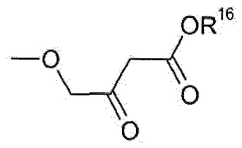 dans laquelle
R¹⁵ est C₁- à C₁₀-alkylène, ou arylène substitué ou non substitué,
R¹⁶ est C₁- à C₁₀-alkyle qui est linéaire ou ramifié, ou aryle substitué ou non substitué ; et
B4) éventuellement d'autres monomères qui sont copolymérisables avec les monomères susmentionnés choisis parmi
B41) des monomères polaires, préférablement le nitrile (méth)acrylique et/ou le (méth)acrylate de méthyle ; et/ou
B42) des monomères non polaires, préférablement le styrène et/ou l'α-méthylstyrène.

8. Utilisation selon l'une quelconque des revendications 1 à 4, 6 ou 7, dans laquelle l'agent anti-salissures comprend un agent fixateur (d).

9. Utilisation selon l'une quelconque des revendications 1 à 8, dans laquelle le matériau textile traité se trouve sous la forme d'un filet.

10. Utilisation selon la revendication 9, dans laquelle le filet possède une taille de maille allant de 2 mm à 30 mm.

11. Utilisation selon la revendication 9 ou 10, dans laquelle les mailles ont une forme tétragonale, hexagonale ou octogonale.

12. Utilisation selon l'une quelconque des revendication 1 à 11, dans laquelle le matériau textile traité comprend, outre le biocide (a), un parasiticide choisi parmi les pyréthrinoïdes, les composés agonistes/antagonistes du récepteur nicotinique, les composés organophosphorés et les insecticides à base de lactone macrocyclique.

13. Utilisation selon la revendication 12 en aquaculture, afin de protéger les animaux en culture contre les parasites.
